# EUROPEAN PATENT SPECIFICATION

(11) **EP 2 890 816 B1**
(45) Date of publication and mention of the grant of the patent: **05.06.2019**
(21) Application number: 13834143.3
(22) Date of filing: 30.08.2013
(51) Int. Cl.: C12Q 1/68, G01N 33/53, G01N 33/567

(54) **PAPP-A2 AS A MARKER FOR MONITORING, PREDICTING AND DIAGNOSING PREECLAMPSIA**
PAPP-A2 ALS MARKER ZUR ÜBERWACHUNG, PROGNOSE UND DIAGNOSE VON PRÄEKLAMPSIE
PAPP-A2 EN TANT QUE MARQUEUR POUR LA SURVEILLANCE, LA PRÉDICTION ET LE DIAGNOSTIC DE LA PRÉÉCLAMPSIE

(30) Priority: 30.08.2012 US 201261694991 P
(43) Date of publication of application: 08.07.2015
(73) Proprietor: Ansh Labs LLC, Webster, TX 77598 (US)
(72) Inventor: KUMAR, Ajay, Friendswood, TX 77546 (US); OXVIG, Claus, DK-8260 VIBY J Stavtrup (DK); SAVJANI, Gopal, V., El Lago, TX 77586 (US)
(74) Representative: Aamand, Jesper L.
(86) International application number: PCT/US2013/057581
(87) International publication number: WO 2014/036440

(56) References cited:
- WO-A1-98/02751
- US-A1- 2008 071 151
- US-A1- 2010 016 173
- US-A1- 2010 304 412
- US-A1- 2011 091 920
- DUGOFF L ET AL: "First-trimester maternal serum PAPP-A and free-beta subunit human chorionic gonadotropin concentrations and nuchal translucency are associated with obstetric complications: A population-based screening study (The FASTER Trial)", AMERICAN JOURNAL OF OBSTETRICS & GYNECOLOGY, MOSBY, ST LOUIS, MO, US, vol. 191, no. 4, 1 October 2004 (2004-10-01), pages 1446-1451, XP004621313, ISSN: 0002-9378, DOI: 10.1016/J.AJOG.2004.06.052
- WINN ET AL.: 'Severe Preeclampsia-Related Changes in Gene Expression at the Matemal-Fetal Interface Include Sialic Acid-Binding Immunoglobulin-Like Lectin-6 and Pappalysin-2' ENDOCRINOLOGY vol. 150, no. 1, January 2009, pages 452 - 462, XP055236392

## Description

### BACKGROUND

Preeclampsia is a medical condition in which hypertension arises in pregnancy in association with significant amounts of protein in the urine. Pre-eclampsia is a condition defined by a set of symptoms rather than any single causative factor, and there are many different causes for the condition. While blood pressure elevation is the most visible sign of the disease, it involves generalized damage to the maternal endothelium, kidneys, and liver, with the release of vasoconstrictive factors being secondary to the original damage.

Preeclampsia may develop from 20 weeks gestation, and it is considered early onset before 32 weeks, which is associated with an increased morbidity. Its progress differs among patients; most cases are diagnosed before labor typically would begin. Pre-eclampsia may also occur up to six weeks after delivery. Apart from Caesarean section and induction of labor (and therefore delivery of the placenta), there is no known cure. It is the most common of the dangerous pregnancy complications, and it may affect both the mother and the unborn child.

Today preeclampsia is typically diagnosed when a pregnant woman develops (i) high blood pressure (such as two separate readings taken at least six hours apart of 140 or more in systolic blood pressure and/or 90 or more in diastolic blood pressure) *and* (ii) proteinuria (such as 300 mg or more of protein in a urine sample).

Emerging predictive tests, including blood tests for quantification of the substances placental growth factor (PIGF) and soluble fms-like tyrosine kinase-1 (sFLT-1) in late first or second trimester, appear to predict early-onset preeclampsia. Tests for the vascular endothelial growth factor (VEGF) family proteins do not have sufficient statistical power to accurately predict late-onset preeclampsia, but may be useful in detection of early-onset preeclampsia (Andraweera, P. H.; Dekker, G. A.; Roberts, C. T. (2012). "The vascular endothelial growth factor family in adverse pregnancy outcomes". Human Reproduction Update 18 (4): 436-457). An alternative test involves checking patients' urine for specific cells called podocytes (Catherine M Brown, Vesna D Garovic, Mechanisms and management of hypertension in pregnant women, Curr Hypertens Rep. 2011 Oct ;13 (5):338-46).

### SUMMARY OF THE INVENTION

The invention provides methods for using PAPP-A2 as a marker for monitoring, predicting and diagnosing preeclampsia in pregnant women. The inventors have discovered that PAPP-A2 levels in pregnant women with preeclampsia are higher than PAPP-A2 levels in normal pregnant women. This is especially true for PAPP-A2 levels that are measured later on in the pregnancy. PAPP-A2 levels may be measured early in pregnancy in order to predict the likelihood of the patient having preeclampsia. Preeclampsia may also be diagnosed at later gestational ages when the levels of PAPP-A2 are more pronounced than normal PAPP-A2 levels at the same gestational age. The present invention relates to methods of assessing, predicting and diagnosing preeclampsia as well as kits-of-parts for assessing, predicting and diagnosing preeclampsia.

More specifically the invention relates to a method to aid in evaluating and/or treating a subject suspected of having or developing preeclampsia, the method comprising the steps of:
(a) determining a level of PAPP-A2 in the subject; and
(b) comparing said level to a standard level of PAPP-A2 representing the absence of preeclampsia, wherein the standard level of PAPP-A2 is determined for a gestation time comparable to that of the subject when performing step (a), and wherein the gestation time of the subject, and the gestation time of a population used to determine the standard level value, respectively, are within three weeks of each other; wherein the standard level of PAPP-A2 is determined as a statistical measure obtained from PAPP-A2 levels of a population of subjects that do not have preeclampsia, and wherein the statistical measure is a centile level in the range from about the 90th centile to about the 99th centile; wherein if said level is greater than said standard level the subject is identified as likely to have preeclampsia or to develop preeclampsia, and if said level is not greater than said standard level the subject is identified as not likely to have preeclampsia or not likely to develop preeclampsia.

The level of a PAPP-A2 polypeptide or a PAPP-A2 nucleic acid can be determined and used as the level of PAPP-A2 in the subject as well as the standard level of PAPP-A2.

In a preferred embodiment the level of a PAPP-A2 polypeptide is determined using a PAPP-A2-specific antibody.

An elevated level of PAPP-A2 can be used for monitoring, predicting and/or diagnosing preeclampsia.

In one embodiment an elevated PAPP-A2 level or activity is defined as a measurement of a PAPP-A2 level or activity that is at or above the 90^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 92^{nd} centile compared to the PAPP-A2 level in normal pregnant women at a comparable time of gestation.

In one embodiment an elevated PAPP-A2 level or activity is defined as a measurement of a PAPP-A2 level or activity that is at or above the 94^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 95^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 96^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 97^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 98^{th} centile compared to the PAPP-A2 level in normal pregnant women at a comparable time of gestation.

In one embodiment an elevated PAPP-A2 level or activity is defined as a measurement of a PAPP-A2 level or activity that is at or above the 99^{th} centile compared to the PAPP-A2 level in normal pregnant women at a comparable time of gestation.

In certain embodiments, the standard level of PAPP-A2 is predetermined. In other embodiments, the method of the invention includes the step of determining the standard level of PAPP-A2 using a population of subjects that do not have preeclampsia.

The above-described method can further comprise:
(c) determining a level in the subject of a secondary marker for preeclampsia such as a secondary marker selected from the group consisting of activin A, sEndoglin, PAPP-A, PAPP-A-ProMBP and/or ProMBP-AGT; and
(d) comparing said secondary marker level to a standard level for said secondary marker;
wherein if said secondary marker level is different from - i.e. either greater than or less than - said secondary marker standard level the subject is identified as more likely to have preeclampsia or to develop preeclampsia, and if said secondary marker level is not greater than said secondary marker standard level the subject is identified as less likely to have preeclampsia or to develop preeclampsia.

In one embodiment, the secondary marker is activin A or sEndoglin.

In one embodiment, the secondary marker is activin A.

In one embodiment, the secondary marker is sEndoglin.

In certain embodiments, the levels of two of said secondary markers are determined and compared to respective standard levels of said secondary markers.

In one embodiment, both secondary marker levels are greater than their respective standard levels, and the subject is identified as more likely to have preeclampsia or to develop preeclampsia than if only one of the two determined secondary markers is greater than its standard level.

In one embodiment, both secondary marker levels are not greater than their respective standard levels, and the subject is identified as less likely to have preeclampsia or to develop preeclampsia than if both of the two determined secondary markers is greater than its standard level.

In one embodiment, the two secondary markers are activin A and sEndoglin.

The subject's blood pressure or urinary protein content can also be determined and this analysis can be taken into account when preeclampsia is diagnosed or prognosed. The subject could have had either hypertension or normal blood pressure prior to becoming pregnant, and the subject's medical history in this regard also can be taken into account.

The invention further relates to the use of a kit-of-parts for diagnosing and/or prognosing preeclampsia comprising one or more reagents for determination of the PAPP-A2 level in a biological sample and instructions for use. The one or more reagents for determination of the PAPP-A2 level can comprise, for example, one or more monoclonal and/or polyclonal antibodies or one or more nucleic acids specific for PAPP-A2.

### BRIEF DESCRIPTION OF THE DRAWINGS

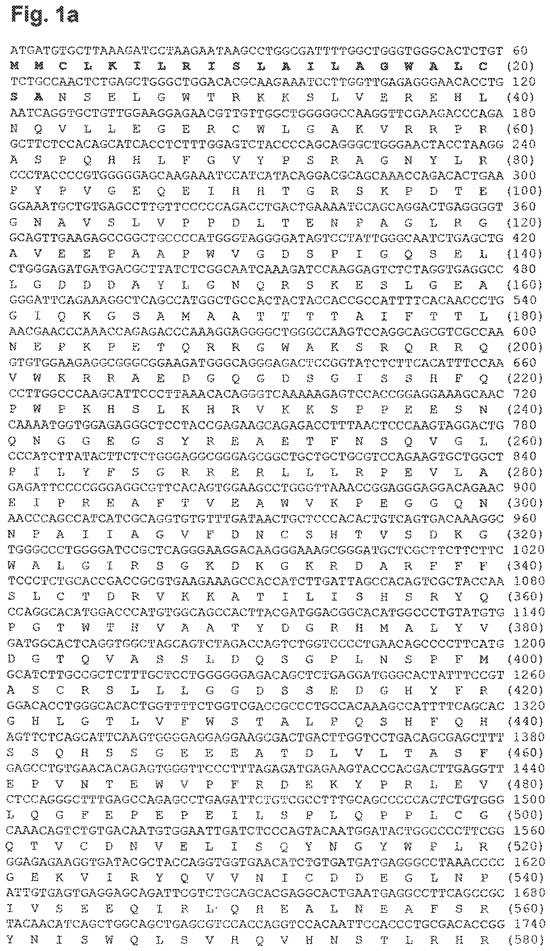

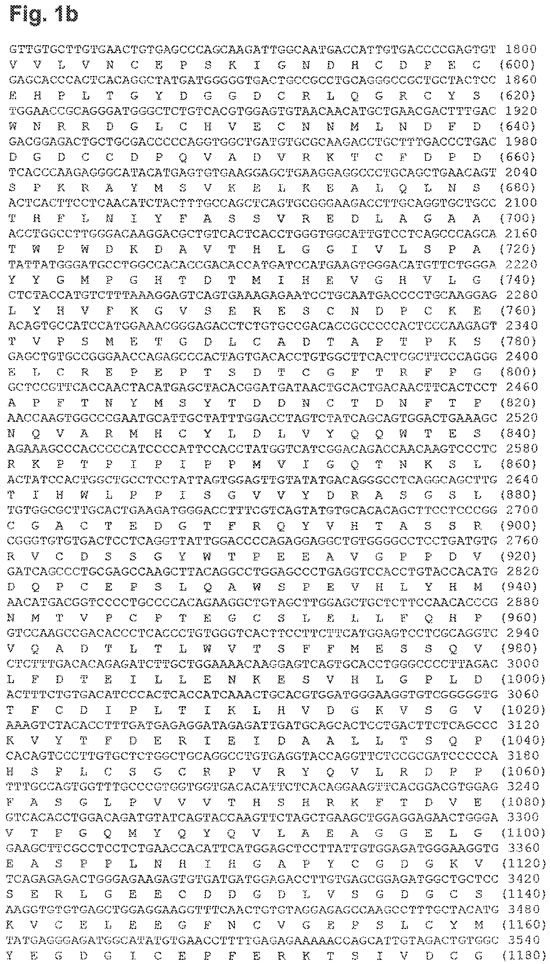

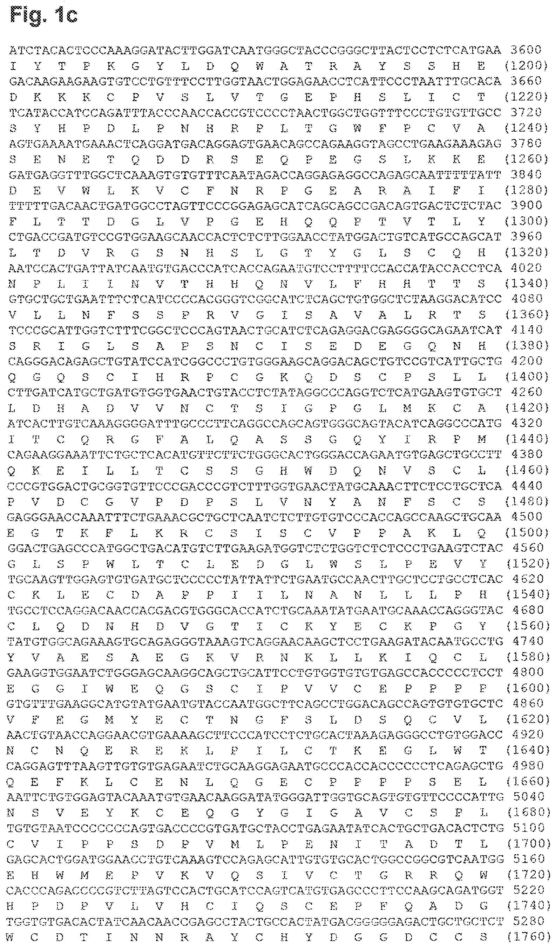

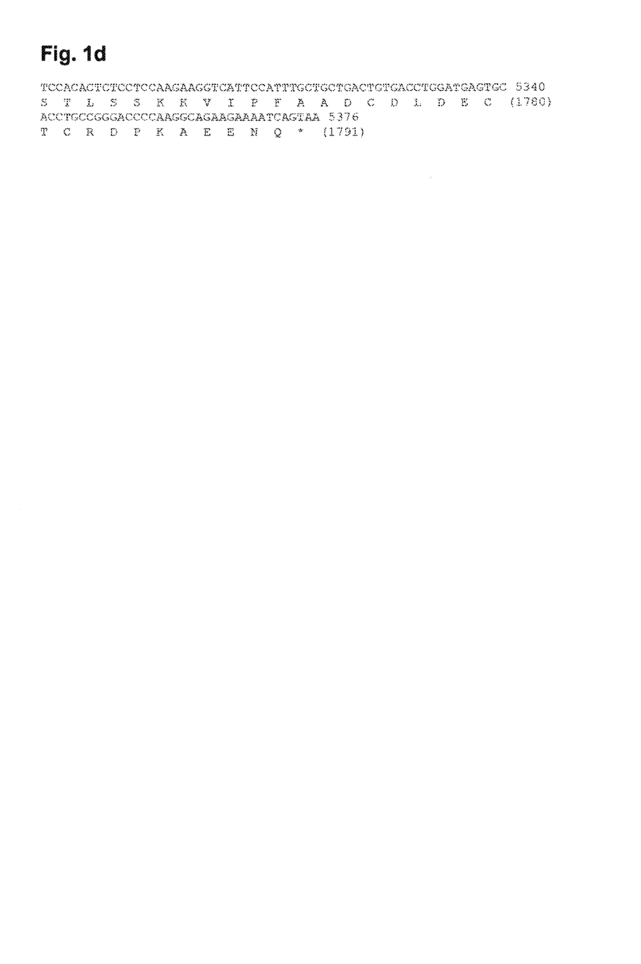

Figure 1 shows PAPP-A2 cDNA and encoded amino acid sequences. Figures 1a - 1d show the cDNA sequence (in 5'→3' orientation) corresponding to the mRNA that encodes preproPAPP-A2. The polypeptide sequence shown is SEQ ID NO:1, and the polynucleotide sequence shown is SEQ ID NO:2. Only the coding part of the sequence and the terminal stop codon (*) are shown and together are numbered 1-5376. The translated polypeptide sequence of preproPAPP-A2 is also shown. The signal peptide cleavage site was predicted using SignalP V2.0 to be after the alanine residue encoded by nt. 64-66 (Nielsen et al., 1997, Protein Eng 10, 1-6). WWW prediction server is located at genome.cbs.dtu.dk. The signal peptide of preproPAPP-A2 (nt. 1-66, 22 residues) is shown in bold.

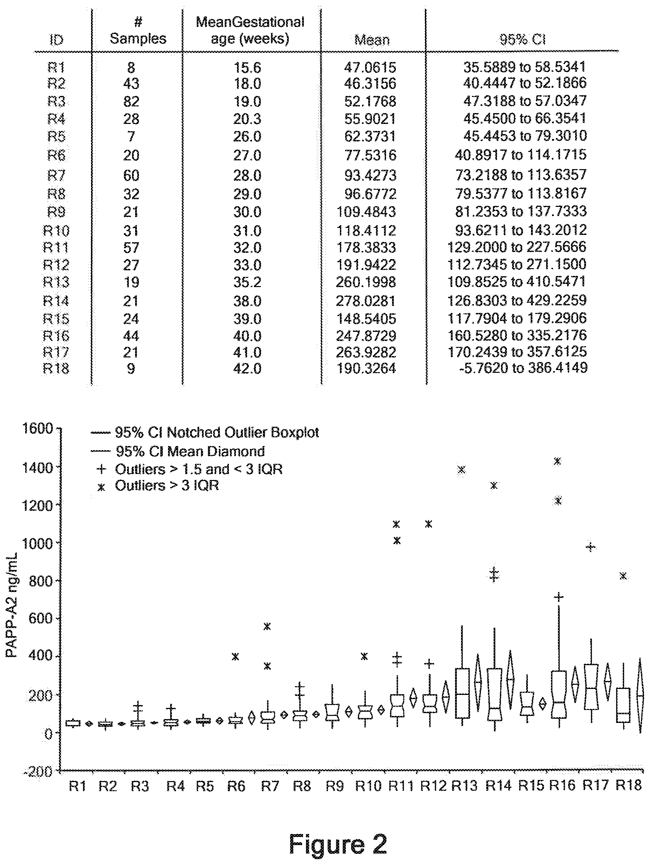

Figure 2 shows PAPP-A2 levels from a variety of samples taken from a population of pregnant women including both women with preeclampsia and women without preeclampsia. Note the gradual increase of PAPP-A2 levels during pregnancy, peaking around 38 gestational weeks. Unlike other biomarkers for preeclampsia (e.g., activin A), normal PAPP-A2 levels during pregnancy gradually increase, rather than sharply increasing late in the pregnancy term.

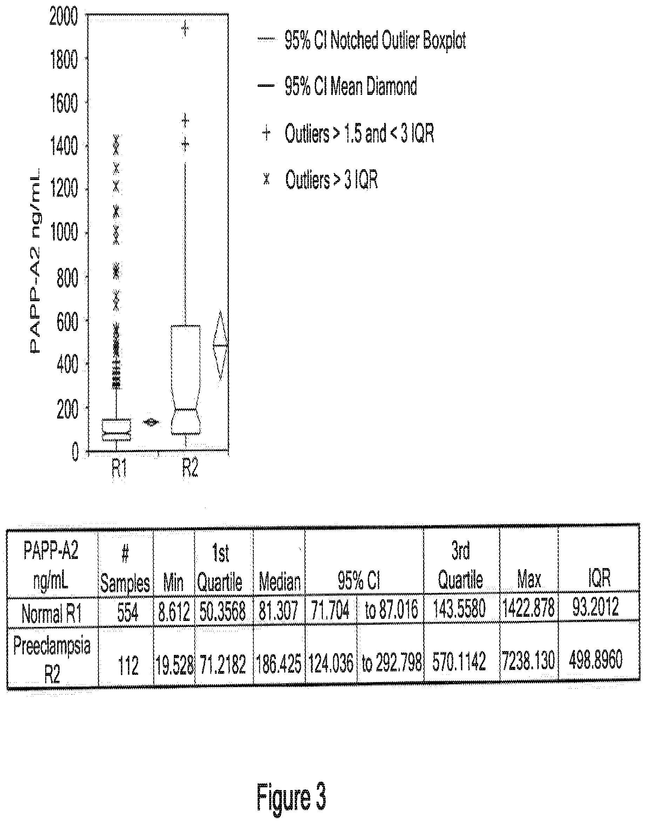

Figure 3 shows PAPP-A2 levels for normal vs. preeclampsia samples from the population of pregnant women shown in Figure 2, at the same average gestational age.The mean levels of PAPP-A2 in ng/ml are shown for each quartile of the normal vs. preeclampsia populations. At the 1^{st} Quartile, normal PAPP-A2 levels were at approximately 50 ng/mL, which corresponds to a gestational age of 19weeks (see Figure 2, which correlates PAPP-A2 levels to gestational age). The first quartile data show a 1.4-fold difference between the values of PAPP-A2 in preeclamptic and normal samples.
   At the 3^{rd} Quartile, normal PAPP-A2 levels were at approximately 143 ng/mL, which corresponds to a gestational age of 32 weeks. There was a 4-fold difference between the values of PAPP-A2 in preeclamptic and normal samples.
   These data demonstrate that while PAPP-A2 is a good marker for preeclampsia throughout pregnancy, the difference in PAPP-A2 values increases exponentially toward the end of gestation in women with preeclampsia. This trend is unlike PAPP-A2 levels in normal women, which gradually increase throughout pregnancy.

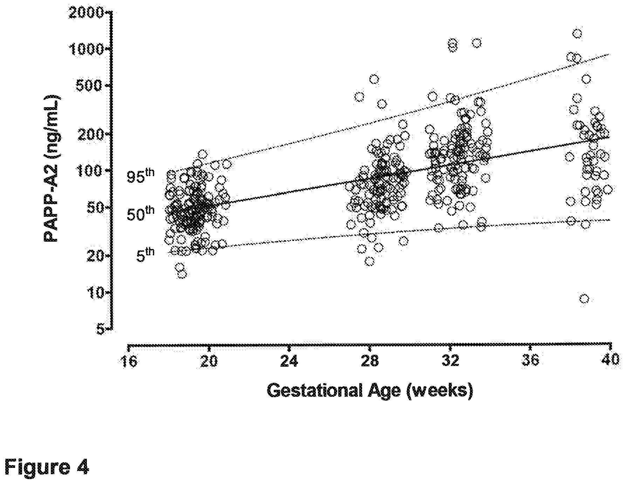

Figure 4 shows PAPP-A2 levels in normal pregnancies stratified into four separate gestational age groupings. Open circles indicate individual patient results. The expected medians (solid lines) along with 5^{th} and 95^{th} centiles (dashed lines) are shown as well. These data provide a gestational age-specific reference set for PAPP-A2 levels from 18 weeks gestation through term.

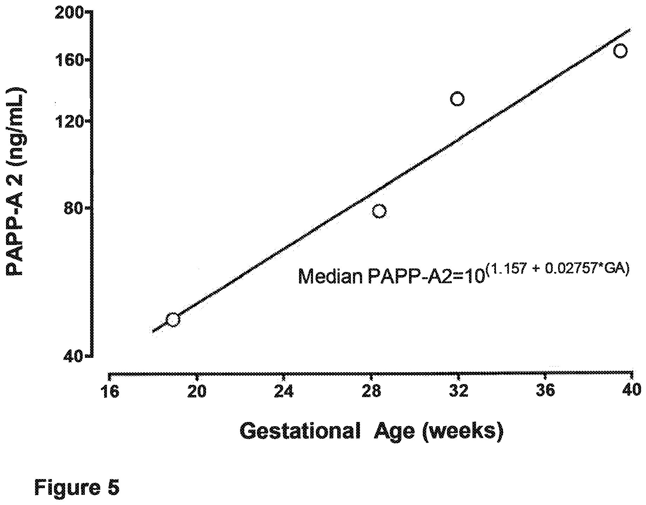

Figure 5 shows regression of the median (log mean) PAPP-A2 values versus gestational age for the data set shown in Figure 4. The median PAPP-A2 and mean gestational ages (open circles) are plotted with the regression line drawn. These data allow for the results to be converted to multiples of the median (MoM).

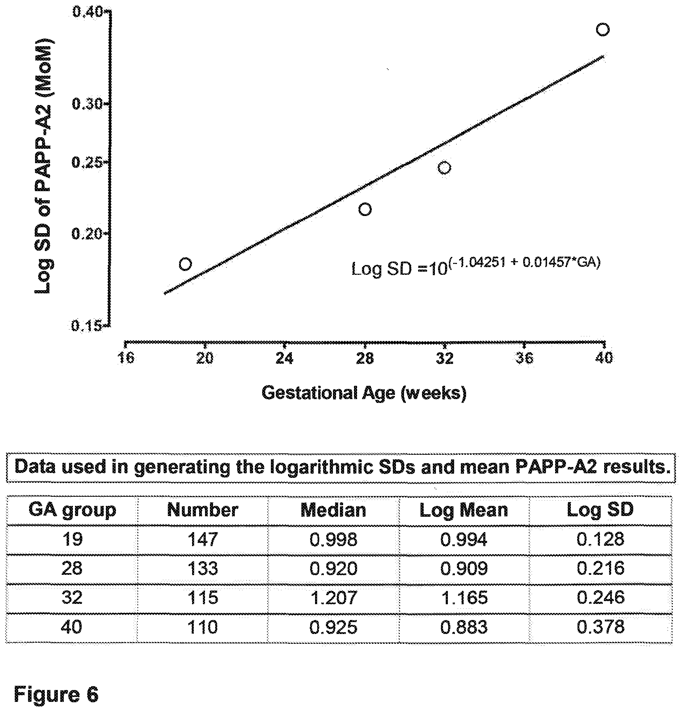

Figure 6 shows regression of the log standard deviation of PAPP-A2 results in MoM, versus gestational age. The log SD of the PAPP-A2 MoM results are plotted versus the mean gestational ages (open circles) with the regression line drawn. These data indicate that the log SD varies considerably during pregnancy.

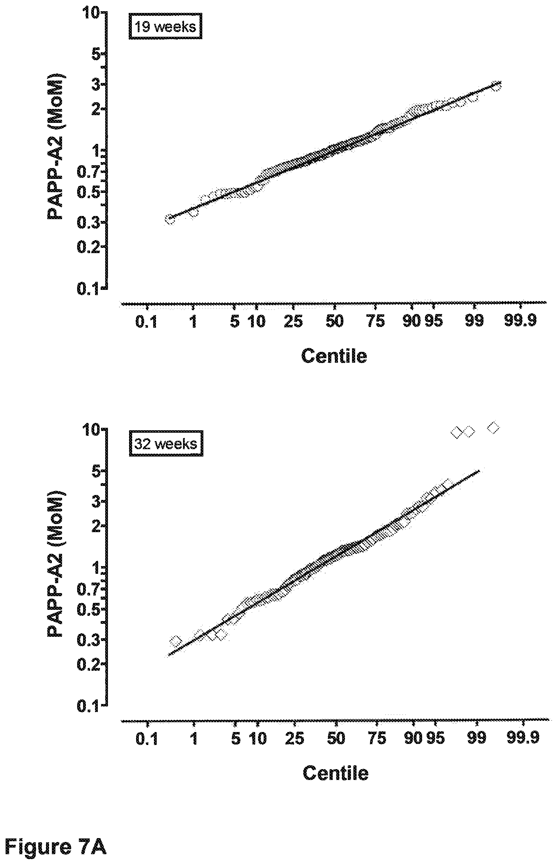

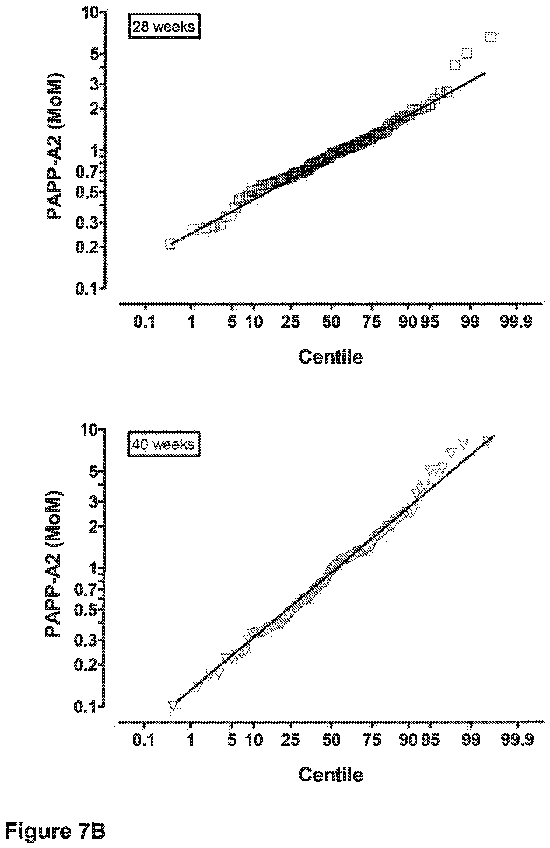

Figure 7 shows individual probability plots for PAPP-A2 values (in MoM) for each of the four gestational age groups. The slope of the fitted line is the logarithmic standard deviation. These estimates are then regressed (Figure 6) to provide estimates of log SD at each week of gestation. Fig. 7A shows plots for 19 and 32 weeks gestation, and Fig. 7B shows plots for 28 and 40 weeks gestation.

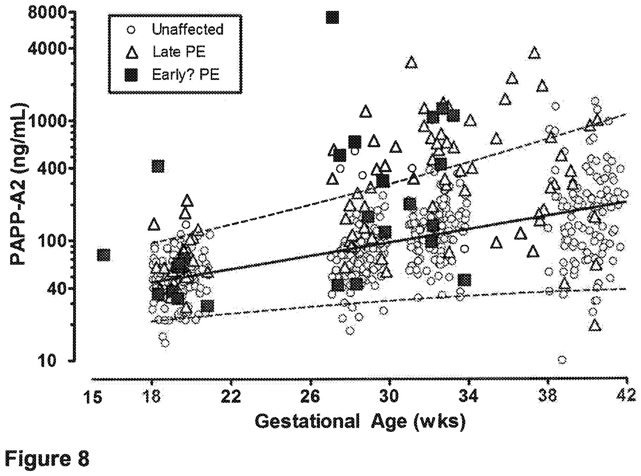

Figure 8 shows PAPP-A2 levels in women who did, or did not, develop preeclampsia, stratified by gestational age. The small open circles indicate observations in women who did not develop preeclampsia. The larger symbols indicate measurements in those who developed preeclampsia, or were preeclamptic at the time of sampling. Triangles indicate samples from women who developed preeclampsia after 34 weeks, indicating that they likely had late preeclampsia. Squares are from women who had no samples noted after 34 weeks. This could indicate severe preeclampsia and early delivery, or that they delivered later, but were not sampled later.

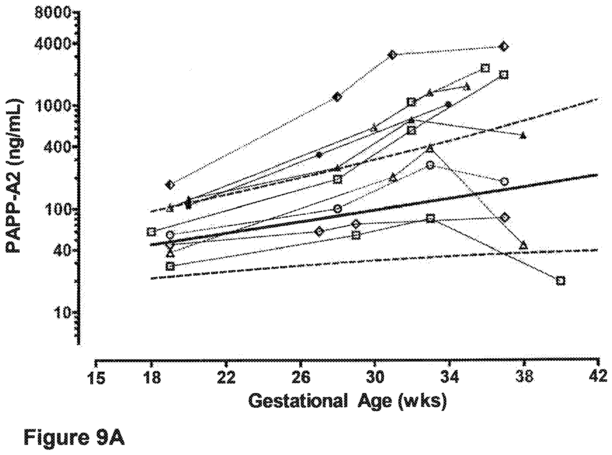

Figures 9A - 9C show linked PAPP-A2 results from three different sets of 10 patients each of whom developed preeclampsia.

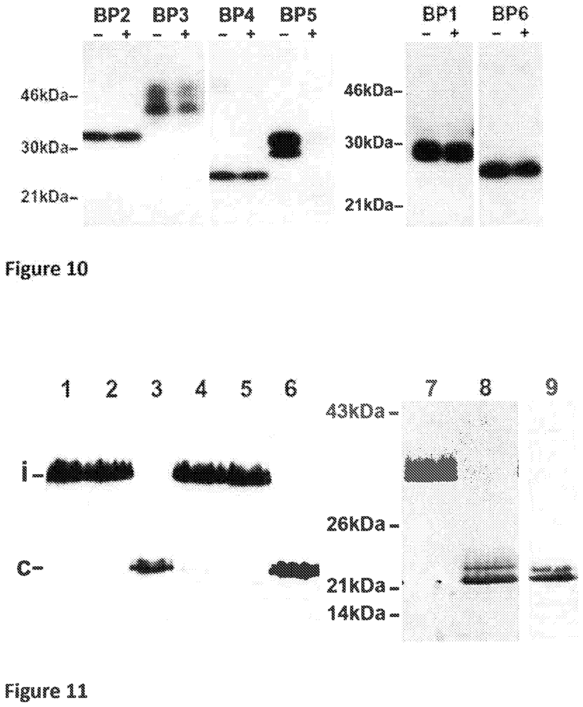

Figure 10 shows the activity of PAPP-A2 against IGFBP-1-6. Medium from 293T cells transfected with empty vector (-), or cDNA encoding PAPP-A2 (pPA2) (+) was incubated with each of the six IGFBPs (BP1-BP6), and the activity was assessed by ligand blotting using radiolabeled IGF-II. Complete cleavage of IGFBP-5 is evident from the absence of a signal in the BP5+ lane. Partial degradation of IGFBP-3 is also evident.
Figure 11 shows proteolytic activity of PAPP-A2 against IGFBP-5. Medium from 293T cells transfected with empty vector (lane 1), cDNA encoding PAPP-A2 with an inactivating E734Q mutation (pPA2-KO) (lane 2), or cDNA encoding wild-type PAPP-A2 (pPA2) (lanes 3-6) was incubated with C-terminally c-myc tagged rIGFBP-5. Proteolytic activity was assessed by Western blotting using anti-c-myc. 'i' denotes intact rIGFBP-5; 'c' denotes the detectable C-terminal c-myc tagged cleavage product. In the absence of inhibitors, wild-type PAPP-A2 degraded all rIGFBP-5 (lane 3). The PAPP-A2 activity was abolished by 10 mM phenanthroline (lane 4) and 5 mM EDTA (lane 5), but not affected by 100 µM 3,4-DCI (lane 6). Coomassie-stained SDS-PAGE of purified rIGFBP-5 is shown before (lane 7) and after (lane 8) digestion with purified PAPP-A2. A Western blot of the same digest, using anti-c-myc, is also shown (lane 9). Sequence analysis revealed that PAPP-A2 cleaves IGFBP-5 at one site, between Ser-142 and Lys-143.

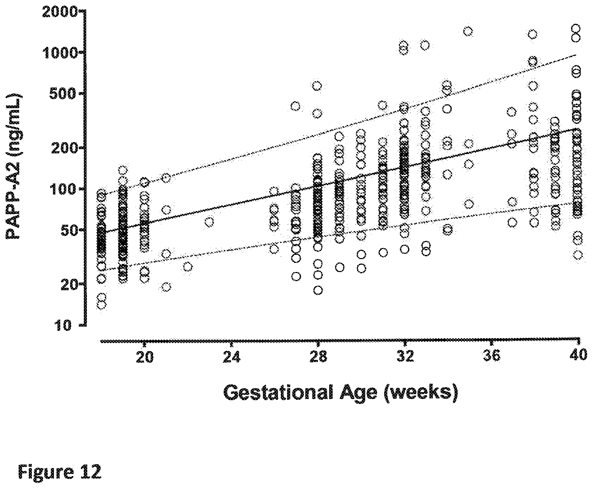

Figure 12 shows control data, i.e., data from women not diagnosed with preeclampsia at any time during pregnancy and associated 5^{th}, 50^{th} and 95^{th} centiles. The median (solid middle line) as well as 90% prediction limits (95^{th} and 5^{th} centiles) are shown (dashed lines). The early second trimester (approximately 18 to 20 completed weeks' gestation) included 161 observations (7 positive or 4.3% positive), the early third trimester (approximately 27 to 33 weeks' gestation) included 254 observations (11 positive or 4.7% positive) and near term (38 to 40 weeks' gestation) included 100 observations (5 positive or 5.0% positive).

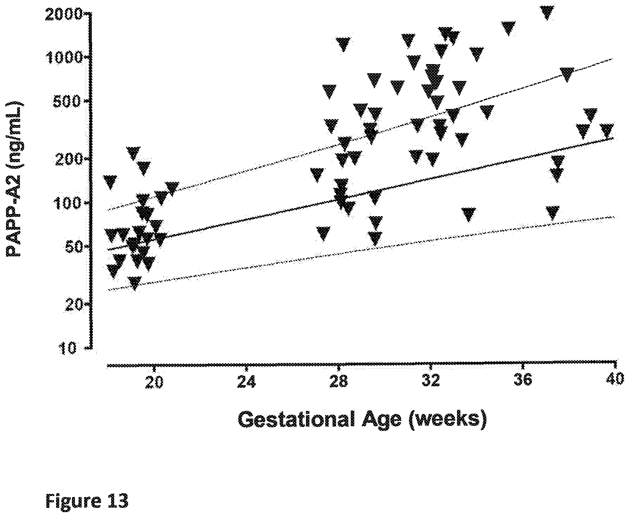

Figure 13 shows the PAPP-A2 levels by gestational age in pregnancies identified in women with mild preeclampsia. In the first gestational age group (approximately 18 to 20 completed weeks' gestation) 5 of the 22 observations in cases (23%, the detection rate) are at or above the 95^{th} centile (false positive rate of 5%). In the early third trimester (approximately 27 to 33 weeks' gestation) this rate increases to 23 of 43 (53% detection). Among the 8 near term cases (38 to 40 weeks' gestation) only two were elevated (25% detection).

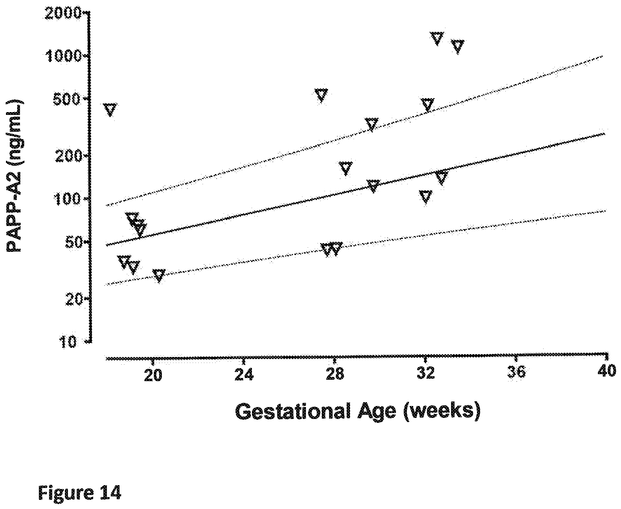

Figure 14 shows the PAPP-A2 levels by gestational age in pregnancies identified with severe preeclampsia. In the first gestational age group (approximately 18 to 20 completed weeks' gestation) only 1 of the 7 observations in cases (14%, the detection rate) is at or above the 95^{th} centile (false positive rate of 5%). In the early third trimester (approximately 27 to 33 weeks' gestation) this rate increases to 5 of 11 (45% detection).

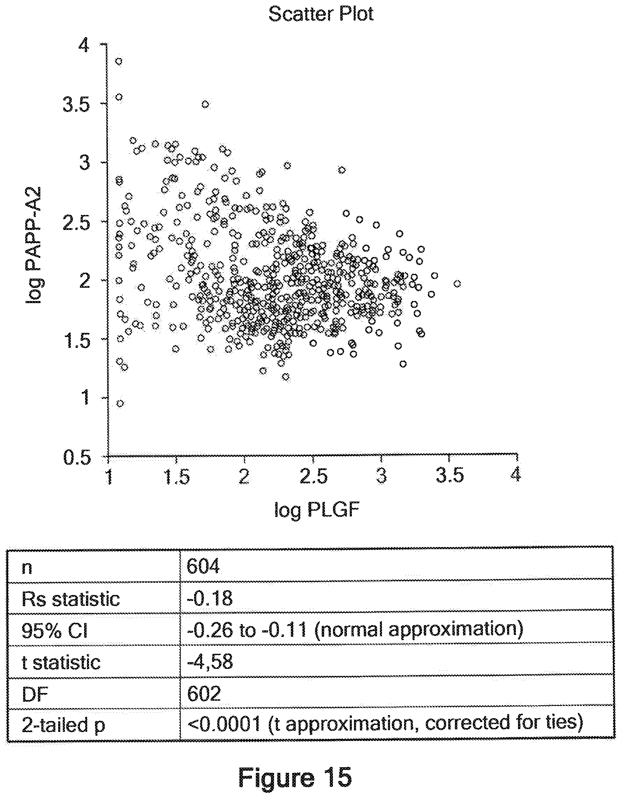

Figure 15 shows a Spearman rank correlation between PLGF and PAPP-A2 when studied on 604 pregnancy serum samples. A negative correlation coefficient of -0.18 with a 2-tailed p of <0.0001 was found.

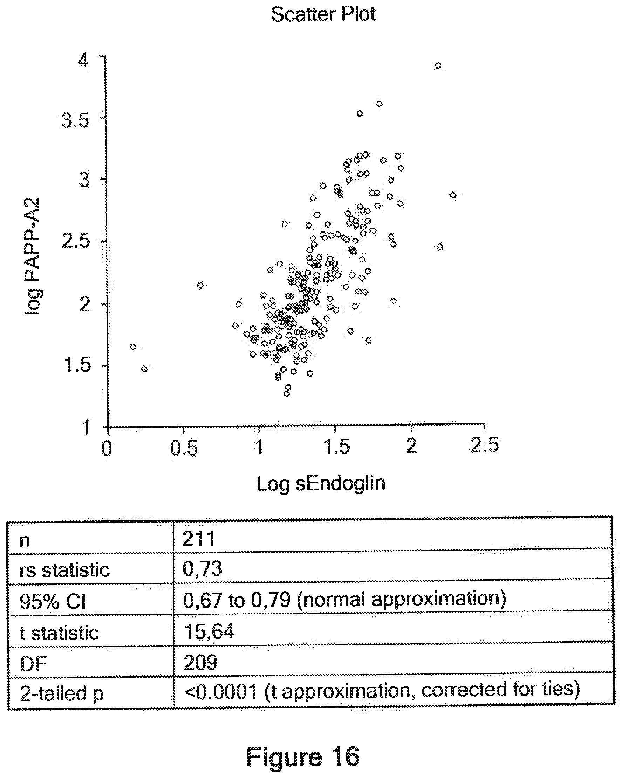

Figure 16 shows a Spearman rank correlation between sEndoglin and PAPP-A2 when studied on 211 pregnancy serum samples. A strong correlation (rs= 0.73, p of <0.0001) was found.

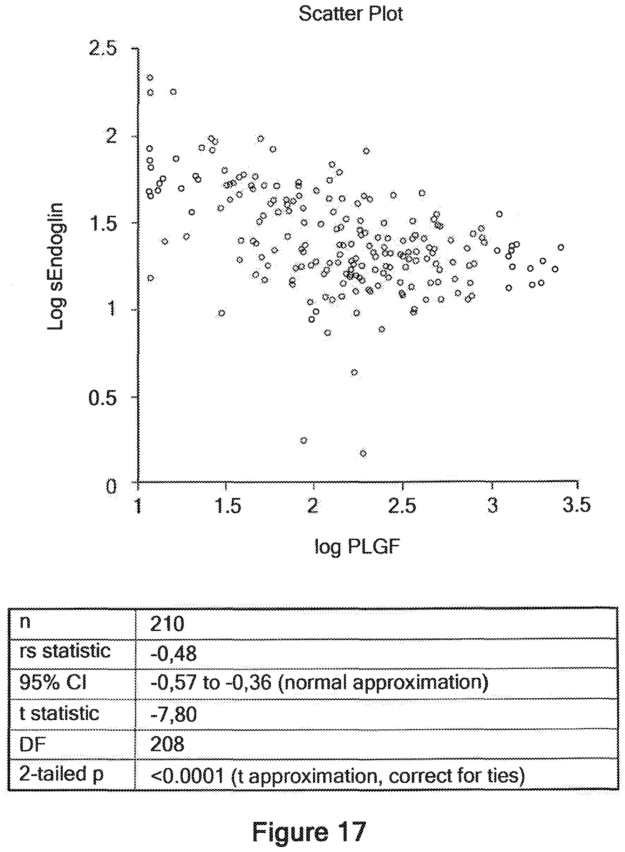

Figure 17 shows a Spearman rank correlation between PLGF and sEndoglin when studied on 210 pregnancy serum samples. A negative correlation coefficient of -0.48 with a 2-25 tailed p of <0.0001 was found.

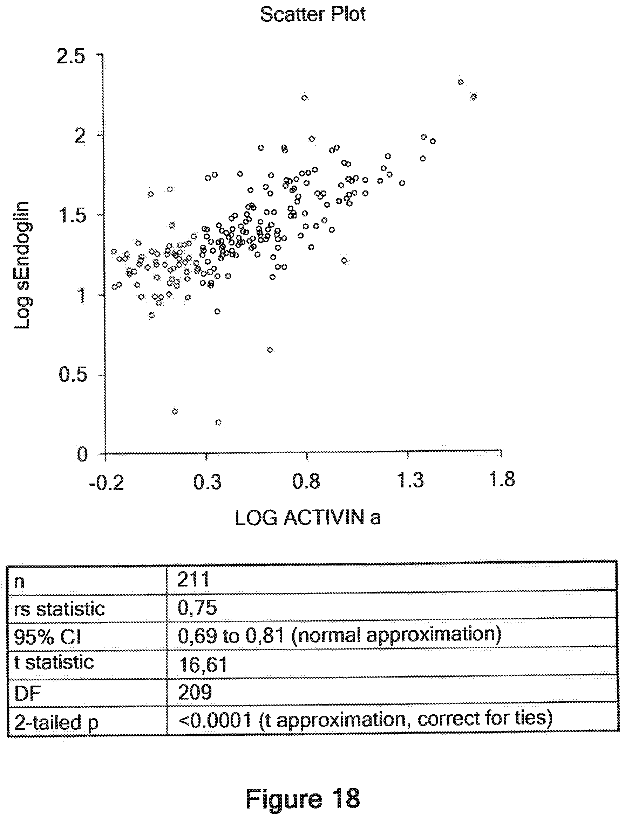

Figure 18 shows a Spearman rank correlation between sEndoglin and Activin A when studied on 211 pregnancy serum samples. A strong correlation (rs= 0.75, p of <0.0001) was found.

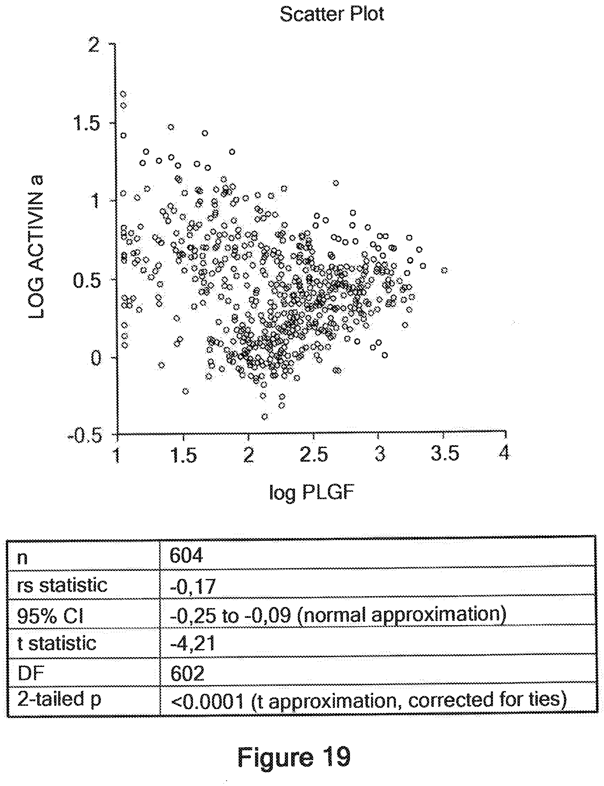

Figure 19 shows a Spearman rank correlation between Activin A and PLGF when studied on 604 pregnancy serum samples. A negative correlation coefficient of -0.17 with a 2-tailed p of <0.0001 was found.

### DETAILED DESCRIPTION OF THE INVENTION

### Definitions

"Preeclampsia": Preeclampsia is a disorder in pregnant women of widespread vascular endothelial malfunction and vasospasm that usually occurs after 20 weeks gestation and can present as late as 4-6 weeks postpartum. It is clinically defined by hypertension and proteinuria, with or without pathologic edema. Medical consensus is lacking regarding the values that define preeclampsia; however, as used herein, "preeclampsia" is present in a woman who was normotensive before 20 weeks' gestation but develops a systolic blood pressure (SBP) greater than 140 mm Hg and/or a diastolic BP (DBP) greater than 90 mm Hg on 2 successive measurements, taken 4-6 hours apart. In a patient with preexisting essential hypertension (i.e., essential hypertension present before the pregnancy), preeclampsia is present if SBP has increased by 30 mm Hg or if DBP has increased by 15 mm Hg. Preeclampsia is often divided into mild and severe preeclampsia. Preeclampsia is mild in 75% of cases and severe in 25% of them. In its extreme, the disease may lead to liver and renal failure, disseminated intravascular coagulopathy (DIC), and central nervous system (CNS) abnormalities. A woman "having preeclampsia" has the sympoms of hypertension described above and may or may not also have proteinuria and may or may not have pathologic edema.

A woman who is "developing preeclampsia" refers to a woman who did not have preeclampsia as defined above in an earlier stage of pregnancy but whose blood pressure is rising into the range characteristic of preeclampsia as defined above. Additional symptoms associated with preeclampsia such as proteinuria and pathologic edema may or may not be present in a woman who is developing preeclampsia.

"Mild preeclampsia": Mild preeclampsia is defined as the presence of hypertension (BP ≥140 mm Hg systolic and/or 90 mm Hg diastolic) on 2 occasions, taken at least 6 hours apart, but without evidence of end-organ damage in the patient.

"Severe preeclampsia": Severe preeclampsia is defined as the presence of 1 of the following symptoms or signs in addition to the presence of preeclampsia:
- SBP of 160 mm Hg or higher or DBP of 110 mm Hg or higher on 2 occasions at least 6 hours apart
- Proteinuria of more than 5 g in a 24-hour collection or more than 3g in 2 random urine samples collected at least 4 hours apart
- Pulmonary edema or cyanosis
- Oliguria (< 400 mL in 24 h)
- Persistent headaches
- Epigastric pain and/or impaired liver function
- Thrombocytopenia
- Oligohydramnios, decreased fetal growth, or placental abruption

"Eclampsia": If preeclampsia-associated seizures develop, the disorder has developed into the condition called eclampsia.

"PAPP-A2 polypeptide and PAPP-A2 polynucleotide": As used herein, PAPP-A2 polypeptide refers to an isolated PAPP-A2 polypeptide having the amino acid sequence listed in Fig. 1 (SEQ ID NO:1) or a variant or fragment thereof as defined herein below or as defined in US Patent No. 7,083,940. A PAPP-A2 encoding nucleotide sequence refers to an isolated nucleic acid having the sequence listed in Fig. 1 (SEQ ID NO:2), or a variant or fragment thereof as defined herein below or as defined in US7,083,940. The content of US7,083,940 is hereby incorporated in its entirety.

The amino acid sequence of PAPP-A2 is composed of a 233-residue pre-pro-piece and a 1558-residue mature portion. In preferred embodiments the of methods of the invention, the PAPP-A2 polypeptide used is the mature portion of PAPP-A2 - i.e. amino acids 234 to 1791 of SEQ ID NO:1 (cf. Fig 1).

"Active PAPP-A2" refers to those PAPP-A2 polypeptides which retain the biological and/or immunological activities of any naturally occurring PAPP-A2.

"Naturally occurring PAPP-A2" refers to PAPP-A2 produced by human cells that have not been genetically engineered and specifically contemplates various PAPP-A2s arising from post-translational modifications of the polypeptide including but not limited to acetylation, carboxylation, glycosylation, phosphorylation, lipidation, acylation, or complex formation, covalent or noncovalent, with other polypeptides.

"Derivative PAPP-A2" refers to polypeptides derived from naturally occurring PAPP-A2 by chemical modifications such as ubiquitination, labeling (e.g., with radionuclides, any of various enzymes, etc.), pegylation (derivatization with polyethylene glycol), or by insertion (or substitution by chemical synthesis) of amino acids such as ornithine, which do not normally occur in human proteins.

"Recombinant variant PAPP-A2" refers to any polypeptide differing from naturally occurring PAPP-A2 by amino acid insertions, deletions, and substitutions, created using recombinant DNA techniques. Guidance in determining which amino acid residues may be replaced, added or deleted without abolishing activities of interest, such as proteolytic activity or cell adhesion, may be found e.g. by comparing parts of the sequence of PAPP-A2 with structurally similar proteins (e.g. other metzincin family proteinases), with locally homologous proteins of known disulfide structure, or by secondary structure predictions.

A PAPP-A2 polypeptide "fragment", "portion", or "segment" is a stretch of amino acid residues of at least about 5 amino acids, often at least about 7 amino acids, typically at least about 9 to 13 amino acids, such as at least about 17 or more amino acids in various embodiments. It may also be a longer stretch of residues up to intact PAPP-A2 in length. To be active, any PAPP-A2 polypeptide or PAPP-A2 polypeptide fragment must have sufficient length to display biologic and/or immunologic activity on their own, or when conjugated to a carrier protein such as keyhole limpet hemocyanin.

A PAPP-A2 polypeptide can also refer to the mature PAPP-A2 sequence (amino acid residues 234 to 1791 of SEQ ID NO:1). PAPP-A2 polypeptides also include polypeptides arising from alternative processing in tissue, sera or body fluids other than the source from which the processed PAPP-A2 was originally isolated. Additionally preferred PAPP-A2 fragments are those which comprise or consist essentially of amino acid residues 1 to 233 of SEQ ID NO:1, corresponding to the prepro part of PAPP-A2; of amino acid residues 23 to 233 of SEQ ID NO:1, corresponding to the pro part of PAPP-A2; of amino acid residues 1 to 22 of SEQ ID NO:1, corresponding to the signal peptide or leader sequence of PAPP-A2; and to such sequences operably linked to the mature part of PAPP-A2 corresponding to amino acid residues 234 to 1791 of SEQ ID NO:1.

"PAPP-A2 antibodies": The term "antibody" as used herein includes both polyclonal and monoclonal antibodies, as well as fragments thereof, such as, Fv, Fab and F(ab)2 fragments that are capable of binding antigen or hapten. It includes conventional murine monoclonal antibodies as well as human antibodies, and humanized forms of non-human antibodies, and it also includes 'antibodies' isolated from phage antibody libraries and synthetic or recombinant antibodies. Preparation of PAPP-A2 antibodies has been described in US7,083,940.

The expression "to aid in evaluating and/or treating" a subject suspected of having preeclampsia or being at increased risk of developing preeclampsia means that the method referred to will assist in determining, in any manner and to any extent, whether the subject has preeclampsia or has an increased risk of developing preeclampsia, or will assist in treating, in any manner and to any extent, a subject who has or who develops preeclampsia. Accordingly, if the method calls for the measurement of PAPP-A2 level in a sample from the subject, then the PAPP-A2 level might not be the only diagnostic or prognostic parameter that is taken into account. The measurement of the level of PAPP-A2 can e.g. reduce the number of false positives and/or the number of false negatives obtained by other diagnostic methods disclosed in the prior art. Thus, aiding in evaluating and/or treating does not require that a 100% accurate diagnosis or prognosis is obtained as a result of the method, or that the condition of preeclampsia is treated with 100% efficacy as a result of the method.

"Determining" the level of PAPP-A2 means detection or measurement of the level of PAPP-A2 protein, the level of PAPP-A2 nucleotide and/or the PAPP-A2 protein or nucleotide activity present in a sample. Any suitable detection method or measurement can be used. The measurement can be qualitative, semi-quantitative and/or quantitative.

The expression "the level of PAPP-A2" means the amount, concentration, or activity of PAPP-A2 protein, or any variant or fragment thereof, or the amount, concentration, or activity of PAPP-A2 nucleotide, or any variant or fragment thereof in a sample.

"Subject" means a pregnant female, preferably a pregnant female human being.

"Absence of preeclampsia" is a condition found in a pregnant woman who does not exhibit the symptoms of preeclampsia as defined herein above.

"Normal level of PAPP-A2" means a level of PAPP-A2 in a population of pregnant women without preeclampsia or an identified risk of developing preeclampsia. The normal level of PAPP-A2 depends on the gestation time. Accordingly, the normal level has to be determined for each respective gestation time period, such as each week or range of weeks during the pregnancy. For example, the mean value or median value of PAPP-A2 level in a population of individuals without preeclampsia or an identified risk of developing preeclampsia can be used as the normal level of PAPP-A2 for comparison to a subject suspected of having or being at risk for developing preeclampsia.

"Standard level of PAPP-A2" means a selected or identified level of PAPP-A2 that represents a particular status with respect to the diagnosis, prognosis, or treatment of preeclampsia at a respective gestation time period. For example, a "standard level of PAPP-A2 representing the absence of preeclampsia" indicates a threshold value of PAPP-A2 above which a subject is diagnosed as having preeclampsia or prognosed as having an increased risk of developing preeclampsia or identified as requiring treatment for preeclampsia. A standard level can be given as a fixed value or as an interval. A standard level can be determined or selected using statistical analysis of PAPP-A2 levels in a population. For example, the standard level for a certain gestation week can be selected by multiplying the normal level of PAPP-A2 in a certain gestation week with a "margin factor(x)". X can be, e.g., 1.1, 1.2, 1.3, 1.4, 1.5, 1.6, 1.7, 1.8, 1.9, 2. 2.5, 3, 3.5, 4, 4.5, 5, 6, 7, 8, 9, 10 or more than 10. In another example, the standard level can be selected as a particular centile level of the population data for subjects used to determine the normal level of PAPP-A2. A centile level of 95% can be chosen as the standard level, for example. In yet another example, the standard level can be selected as a particular confidence level for the distribution of PAPP-A2 levels in a population of normal subjects. If the subject has a level of PAPP-A2 above the standard level in the same gestation week or a comparable gestation period, this can be taken as an indication of the existence of preeclampsia or a risk of development of preeclampsia, depending on the particular method.

"Comparable" gestation time refers to a gestation time for a subject's level of PAPP-A2 that is in the same gestation period, such as the same week or interval of weeks, as the gestation time for which the standard PAPP-A2 level is determined. For example, if the subject is tested in gestation week 32, then a comparable gestation time for determining the standard level or the normal level of PAPP-A2 might be any time within week 32, or within weeks 31-33, depending on the particular method. Similarly, population data for determination of a normal or standard level of PAPP-A2 can be combined for subjects within a comparable gestation period, such as within the same week or group of 1, 2, or 3 weeks.

A subject is considered to be "likely to have preeclampsia" if, as the outcome of a method of the present invention, the subject deviates from the standard level of PAPP-A2 according to the particular method. This refers to any increase in likelihood or probability to have preeclampsia compared to a subject did not deviate from the standard level. Which degree of likelihood to have preeclampsia merits treatment or providing certain advice to the subject is generally left to the judgment of a medical professional utilizing the method. The likelihood of having preeclampsia as determined by a method of the invention can be a qualitative assessment, such as a "yes or no" assessment, or it can be associated with a probability of having preeclampsia determined by statistical analysis of the subject's PAPP-A2 value compared with the PAPP-A2 values of a population of normal subjects at comparable gestation time. Whether a subject is "likely to develop preeclampsia" is a similar determination made for a subject that does not, at the time a sample is collected, have the characteristics of preeclampsia, as defined above. However, the determination of whether a subject is likely to develop preeclampsia can involve different standard or normal levels of PAPP-A2, or levels of PAPP-A2 (for both subject and standard or normal) that are determined at different gestation times, or determined over a range or sequence of gestation times. A subject who has been identified as likely to develop preeclampsia can have any degree of increased likelihood or probability of developing preeclampsia compared to normal subjects at comparable gestation time. Levels of secondary markers different from PAPP-A2 also can be used to determine whether a subject is likely to have or to develop preeclampsia.

"Population of subjects" is a group of pregnant women without preeclampsia or without an identified risk of developing preeclampsia that is used to determine the "standard level" and the "normal level" of PAPP-A2.

"Centile level" or percentile (or centile) is the value of a variable below which a certain percent of observations fall. For example, the 90th centile or percentile of PAPP-A2 levels is the value within a population of normal subjects below which 90 percent of the subjects are found.

"Confidence interval" (CI) is a kind of interval estimate of a population parameter and is used to indicate the reliability of an estimate. It is an observed interval (i.e. it is calculated from the observations), in principle different from sample to sample, that frequently includes the parameter of interest, if the experiment is repeated. How frequently the observed interval contains the parameter is determined by the confidence level or confidence coefficient. More specifically, the meaning of the term "confidence level" is that, if confidence intervals are constructed across many separate data analyses of repeated (and possibly different) experiments, the proportion of such intervals that contain the true value of the parameter will match the confidence level; this is guaranteed by the reasoning underlying the construction of confidence intervals

"Secondary marker for preeclampsia" is any marker other than PAPP-A2 that can be used to determine if a pregnant woman has preeclampsia or an increased risk of developing preeclampsia. The secondary marker can be any marker disclosed in the prior art or disclosed in the present application. The combination of PAPP-A2 levels with the level of one or more secondary markers may increase the reliability of predicting the likelihood of having or developing preeclampsia compared to using the level of PAPP-A2 alone, or it may suggest additional treatment options, such as by identifying underlying or secondary mechanisms affecting the subject.

"More likely" in connection with use of a secondary marker for diagnosing or prognosing preeclampsia means that if both PAPP-A2 and the secondary marker give positive results, the diagnosis and/or prognosis of preeclampsia is more reliable or more likely to be correct. This can mean in one embodiment that the number of false positives is reduced by e.g. more than 5%, more than 10%, more than 20%, more than 30%, more than 40%, more than 50%, more than 60%, more than 70%, more than 80 or even more than 90% compared to a method in which PAPP-A2 or the secondary marker was used as the only marker. This can mean in another embodiment that the number of false negatives is reduced by e.g. more than 5%, more than 10%, more than 20%, more than 30%, more than 40%, more than 50%, more than 60%, more than 70%, more than 80 or even more than 90% compared to a method in which PAPP-A2 or the secondary marker was used as the only marker.

"Diagnosed" as having preeclampsia means that it has been determined, with some probability or margin of error, which may be defined or unknown, that the subject has preeclampsia.

"Prognosed" as likely to develop preeclampsia means that it has been determined, with some probablility or margin of error, which may be defined or unknown, that the subject has a predisposition to have or will have preeclampsia at a later gestation time during the pregnancy, although the subject most likely does not have preeclampsia at the time the prognosis is made.

"Hypertension" (HTN) or high blood pressure is a medical condition in which the blood pressure in the arteries is elevated. Blood pressure is summarised by two measurements, systolic and diastolic. Normal blood pressure at rest can be defined as within the range of 100-140mmHg for systolic pressure (top reading) and 60-90mmHg for diastolic pressure (bottom reading). High blood pressure is said to be present if the blood pressure is persistently at or above 140/90 mmHg.

### Method for assessing, predicting and diagnosing preeclampsia

The present invention relates to a method for monitoring, predicting and/or diagnosing the clinical condition preeclampsia during pregnancy. The method comprises measurement of the PAPP-A2 level in the pregnant woman.

In one aspect of the disclosure there is provided a method for detecting PAPP-A2, or measuring the level of PAPP-A2, in a biological sample obtained from an individual (i.e., a subject) in order to assess, predict and/or diagnose preeclampsia, said method comprising the steps of
i) obtaining a biological sample from said individual,
ii) detecting PAPP-A2 in said sample by detecting
   a) a PAPP-A2 polypeptide, or a fragment thereof, and/or
   b) a polynucleotide in the form of mRNA originating from PAPP-A2 expression, and/or
   c) PAPP-A2 specific protease activity, preferably IGFBP-5 protease activity, or proteolytic activity directed against a derivative of IGFBP-5,

The method may comprise the further step of comparing the PAPP-A2 or the level of PAPP-A2 detected in step ii) with a predetermined value selected from the group consisting of
a) a predetermined amount and/or concentration of PAPP-A2; and/or
b) a predetermined amount and/or concentration of PAPP-A2 mRNA; and/or
c) a predetermined PAPP-A2 specific protease activity.

"Active PAPP-A2" and/or "Naturally occurring PAPP-A2" and/or "Derivative PAPP-A2" and/or "mature PAPP-A2" can e.g. be measured.

Different levels of PAPP-A2 in healthy women during pregnancy and in women suffering from, or at risk of contracting, preeclampsia during pregnancy can be determined. This information is used to determine the "normal PAPP-A2 level" and/or the "standard PAPP-A2 level".

Comparing the PAPPA-A2 level and/or activity of said individual to a "normal level of PAPP-A2" - which can be predetermined e.g. as shown in Figure 2 - can be used for diagnosing or prognosing preeclampsia when the PAPP-A2 level in said individual is outside a given range or above a certain threshold e.g. determined by multiplying a "margin factor (x)" times the mean, the median, or another statistical parameter such as a selected centile value or confidence interval value (e.g., 95% CI) derived from the normal data set for a comparable gestation time.

In one embodiment the margin factor can be 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10 or more than 10 or any interval limited by any two of these values.

The predetermined value (i.e. the normal level of PAPP-A2) can in one embodiment be indicative of a normal physiological condition of said individual.

In one embodiment the normal level of PAPP-A2 protein in a plasma sample from gestation week 15, 16 or 17 can be selected from the group consisting of 35 ng/ml, 36 ng/ml, 37 ng/ml, 38 ng/ml, 39 ng/ml, 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml and 60 ng/ml or any interval limited by any two of said levels.

In one embodiment the normal level of PAPP-A2 protein in a plasma sample from gestation week 18 can be selected from the group consisting of 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml and 55 ng/ml or any interval limited by any two of said levels.

In one embodiment the normal level of PAPP-A2 protein in a plasma sample from gestation week 19 can be selected from the group consisting of 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml and 60 ng/ml or any interval limited by any two of said levels.

In one embodiment the normal level of PAPP-A2 protein in a plasma sample from gestation week 20, 21, 22, 23, 24 or 25 can be selected from the group consisting of 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml and 68 ng/ml or any interval limited by any two of said levels.

In one embodiment the normal level of PAPP-A2 protein in a plasma sample from gestation week 20, 21, 22, 23, 24, 25 or 26 can be selected from the group consisting of 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml and 82 ng/ml or any interval limited by any two of said levels.

In one embodiment the normal level of PAPP-A2 protein in a plasma sample from gestation week 26, 27 and 28 can be selected from the group consisting of 40 ng/ml, 41 ng/ml, 42 ng/ml, 43 ng/ml, 44 ng/ml, 45 ng/ml, 46 ng/ml, 47 ng/ml, 48 ng/ml, 49 ng/ml, 50 ng/ml, 51 ng/ml, 52 ng/ml, 53 ng/ml, 54 ng/ml, 55 ng/ml, 56 ng/ml, 57 ng/ml, 58 ng/ml, 59 ng/ml, 60 ng/ml, 61 ng/ml, 62 ng/ml, 63 ng/ml, 64 ng/ml, 65 ng/ml, 66 ng/ml, 67 ng/ml, 68 ng/ml, 69 ng/ml, 70 ng/ml, 71 ng/ml, 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102, ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml and 116 ng/ml or any interval limited by any two of said levels.

In one embodiment the normal level of PAPP-A2 protein in a plasma sample from gestation week 27, 28 and 29 can be selected from the group consisting of 72 ng/ml, 73 ng/ml, 74 ng/ml, 75 ng/ml, 76 ng/ml, 77 ng/ml, 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102, ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml and 116 ng/ml or any interval limited by any two of said levels.

In one embodiment the normal level of PAPP-A2 protein in a plasma sample from gestation week 28, 29 and 30 can be selected from the group consisting of 78 ng/ml, 79 ng/ml, 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102, ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, and 116 ng/ml or any interval limited by any two of said levels.

In one embodiment the normal level of PAPP-A2 protein in a plasma sample from gestation week 29, 30 and 31 can be selected from the group consisting of 80 ng/ml, 81 ng/ml, 82 ng/ml, 83 ng/ml, 84 ng/ml, 85 ng/ml, 86 ng/ml, 87 ng/ml, 88 ng/ml, 89 ng/ml, 90 ng/ml, 91 ng/ml, 92 ng/ml, 93 ng/ml, 94 ng/ml, 95 ng/ml, 96 ng/ml, 97 ng/ml, 98 ng/ml, 99 ng/ml, 100 ng/ml, 101 ng/ml, 102, ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml and 140 ng/ml or any interval limited by any two of said levels.

In one embodiment the normal level of PAPP-A2 protein in a plasma sample from gestation week 30, 31 and 32 can be selected from the group consisting of 93 ng/ml, 94 ng/ml, 95 ng/ml, 96 ng/ml, 97 ng/ml, 98 ng/ml, 99 ng/ml, 100 ng/ml, 101 ng/ml, 102, ng/ml, 103 ng/ml, 104 ng/ml, 105 ng/ml, 106 ng/ml, 107 ng/ml, 108 ng/ml, 109 ng/ml, 110 ng/ml, 111 ng/ml, 112 ng/ml, 113 ng/ml, 114 ng/ml, 115 ng/ml, 116 ng/ml, 117 ng/ml, 118 ng/ml, 119 ng/ml, 120 ng/ml, 121 ng/ml, 122 ng/ml, 123 ng/ml, 124 ng/ml, 125 ng/ml, 126 ng/ml, 127 ng/ml, 128 ng/ml, 129 ng/ml, 130 ng/ml, 131 ng/ml, 132 ng/ml, 133 ng/ml, 134 ng/ml, 135 ng/ml, 136 ng/ml, 137 ng/ml, 138 ng/ml, 139 ng/ml, 140 ng/ml, 141 ng/ml 142 ng/ml, 143 ng/ml, 144 ng/ml and 145 ng/ml or any interval limited by any two of said levels.

In one embodiment the normal level of PAPP-A2 protein in a plasma sample from gestation week 31, 32 and 33 can be selected from the group consisting of 128 ng/ml, 130 ng/ml, 132 ng/ml, 134 ng/ml, 136 ng/ml, 138 ng/ml, 140 ng/ml, 142 ng/ml, 144 ng/ml, 146 ng/ml, 148 ng/ml, 150 ng/ml, 152 ng/ml, 154 ng/ml, 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml and 230 ng/ml or any interval limited by any two of said levels.

In one embodiment the normal level of PAPP-A2 protein in a plasma sample from gestation week 32, 33 and 34 can be selected from the group consisting of 110 ng/ml, 112 ng/ml, 114 ng/ml, 116 ng/ml, 118 ng/ml, 120 ng/ml, 122 ng/ml, 124 ng/ml, 126 ng/ml, 128 ng/ml, 130 ng/ml, 132 ng/ml, 134 ng/ml, 136 ng/ml, 138 ng/ml, 140 ng/ml, 142 ng/ml, 144 ng/ml, 146 ng/ml, 148 ng/ml, 150 ng/ml, 152 ng/ml, 154 ng/ml, 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, and 276 ng/ml or any interval limited by any two of said levels.

In one embodiment the normal level of PAPP-A2 protein in a plasma sample from gestation week 33, 34, 35, 36 and 37 can be selected from the group consisting of 108 ng/ml, 110 ng/ml, 112 ng/ml, 114 ng/ml, 116 ng/ml, 118 ng/ml, 120 ng/ml, 122 ng/ml, 124 ng/ml, 126 ng/ml, 128 ng/ml, 130 ng/ml, 132 ng/ml, 134 ng/ml, 136 ng/ml, 138 ng/ml, 140 ng/ml, 142 ng/ml, 144 ng/ml, 146 ng/ml, 148 ng/ml, 150 ng/ml, 152 ng/ml, 154 ng/ml, 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, 276 ng/ml, 280 ng/ml, 284 ng/ml, 288 ng/ml, 292 ng/ml, 296 ng/ml, 300 ng/ml, 304 ng/ml, 308 ng/ml, 312 ng/ml, 316 ng/ml, 320 ng/ml, 324 ng/ml, 328 ng/ml, 332 ng/ml, 336 ng/ml, 340 ng/ml, 344 ng/ml, 348 ng/ml, 352 ng/ml, 356 ng/ml, 360 ng/ml, 364 ng/ml, 368 ng/ml, 372 ng/ml, 376 ng/ml, 380 ng/ml, 384 ng/ml, 388 ng/ml, 392 ng/ml, 396 ng/ml, 400 ng/ml, 404 ng/ml, 408 ng/ml, 412 ng/ml, and 416 ng/ml or any interval limited by any two of said levels.

In one embodiment the normal level of PAPP-A2 protein in a plasma sample from gestation week 37, 38 and 39 can be selected from the group consisting of 124 ng/ml, 126 ng/ml, 128 ng/ml, 130 ng/ml, 132 ng/ml, 134 ng/ml, 136 ng/ml, 138 ng/ml, 140 ng/ml, 142 ng/ml, 144 ng/ml, 146 ng/ml, 148 ng/ml, 150 ng/ml, 152 ng/ml, 154 ng/ml, 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, 276 ng/ml, 280 ng/ml, 284 ng/ml, 288 ng/ml, 292 ng/ml, 296 ng/ml, 300 ng/ml, 304 ng/ml, 308 ng/ml, 312 ng/ml, 316 ng/ml, 320 ng/ml, 324 ng/ml, 328 ng/ml, 332 ng/ml, 336 ng/ml, 340 ng/ml, 344 ng/ml, 348 ng/ml, 352 ng/ml, 356 ng/ml, 360 ng/ml, 364 ng/ml, 368 ng/ml, 372 ng/ml, 376 ng/ml, 380 ng/ml, 384 ng/ml, 388 ng/ml, 392 ng/ml, 396 ng/ml, 400 ng/ml, 404 ng/ml, 408 ng/ml, 412 ng/ml, 416 ng/ml, 420 ng/ml, 424 ng/ml, 428 ng/ml, and 432 ng/ml or any interval limited by any two of said levels.

In one embodiment the normal level of PAPP-A2 protein in a plasma sample from gestation week 38, 39 and 40 can be selected from the group consisting of 114 ng/ml, 116 ng/ml, 118 ng/ml, 120 ng/ml, 122 ng/ml, 124 ng/ml, 126 ng/ml, 128 ng/ml, 130 ng/ml, 132 ng/ml, 134 ng/ml, 136 ng/ml, 138 ng/ml, 140 ng/ml, 142 ng/ml, 144 ng/ml, 146 ng/ml, 148 ng/ml, 150 ng/ml, 152 ng/ml, 154 ng/ml, 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, and 180 ng/ml or any interval limited by any two of said levels.

In one embodiment the normal level of PAPP-A2 protein in a plasma sample from gestation week 39, 40 and 41 can be selected from the group consisting of 156 ng/ml, 158 ng/ml, 160 ng/ml, 162 ng/ml, 164 ng/ml, 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, 276 ng/ml, 280 ng/ml, 284 ng/ml, 288 ng/ml, 292 ng/ml, 296 ng/ml, 300 ng/ml, 304 ng/ml, 308 ng/ml, 312 ng/ml, 316 ng/ml, 320 ng/ml, 324 ng/ml, 328 ng/ml, 332 ng/ml, 336 ng/ml, and 340 ng/ml or any interval limited by any two of said levels.

In one embodiment the normal level of PAPP-A2 protein in a plasma sample from gestation week 40, 41 and 42 can be selected from the group consisting of 166 ng/ml, 168 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, 276 ng/ml, 280 ng/ml, 284 ng/ml, 288 ng/ml, 292 ng/ml, 296 ng/ml, 300 ng/ml, 304 ng/ml, 308 ng/ml, 312 ng/ml, 316 ng/ml, 320 ng/ml, 324 ng/ml, 328 ng/ml, 332 ng/ml, 336 ng/ml, 340 ng/ml, 344 ng/ml, 348 ng/ml, 352 ng/ml, 356 ng/ml, 360 ng/ml, 364 ng/ml, and 368 ng/ml or any interval limited by any two of said levels.

In one embodiment the normal level of PAPP-A2 protein in a plasma sample from gestation week 41, 42 and 43 can be selected from the group consisting of 30 ng/ml, 40 ng/ml, 50 ng/ml, 60 ng/ml, 70 ng/ml, 80 ng/ml, 90 ng/ml, 100 ng/ml, 110 ng/ml, 120 ng/ml, 130 ng/ml, 140 ng/ml, 150 ng/ml, 160 ng/ml, 170 ng/ml, 172 ng/ml, 174 ng/ml, 176 ng/ml, 178 ng/ml, 180 ng/ml, 182 ng/ml, 184 ng/ml, 186 ng/ml, 188 ng/ml, 190 ng/ml, 192 ng/ml, 194 ng/ml, 196 ng/ml, 198 ng/ml, 200 ng/ml, 202 ng/ml, 204 ng/ml, 206 ng/ml, 208 ng/ml, 210 ng/ml, 212 ng/ml, 214 ng/ml, 216 ng/ml, 218 ng/ml, 220 ng/ml, 222 ng/ml, 224 ng/ml, 226 ng/ml, 228 ng/ml, 230 ng/ml, 232 ng/ml, 234 ng/ml, 236 ng/ml, 238 ng/ml, 240 ng/ml, 242 ng/ml, 244 ng/ml, 246 ng/ml, 248 ng/ml, 250 ng/ml, 252 ng/ml, 254 ng/ml, 256 ng/ml, 258 ng/ml, 260 ng/ml, 262 ng/ml, 264 ng/ml, 266 ng/ml, 268 ng/ml, 270 ng/ml, 272 ng/ml, 274 ng/ml, 276 ng/ml, 280 ng/ml, 284 ng/ml, 288 ng/ml, 292 ng/ml, 296 ng/ml, 300 ng/ml, 304 ng/ml, 308 ng/ml, 312 ng/ml, 316 ng/ml, 320 ng/ml, 324 ng/ml, 328 ng/ml, 332 ng/ml, 336 ng/ml, 340 ng/ml, 344 ng/ml, 348 ng/ml, 352 ng/ml, 356 ng/ml, 360 ng/ml, 364 ng/ml, 368 ng/ml, 372 ng/ml, 376 ng/ml, 380 ng/ml, 384 ng/ml, 388 ng/ml, and 392 ng/ml or any interval limited by any two of said levels.

The standard level of PAPP-A2 protein in a plasma sample taken in gestation week 19 can be selected from the group consisting of 60 ng/ml, 70 ng/ml, 80 ng/ml, 90 ng/ml, 95 ng/ml, 100 ng/ml, 105 ng/ml, 110 ng/ml, 115 ng/ml and 120 ng/ml or any interval limited by any two of said levels.

The standard level of PAPP-A2 protein in a plasma sample taken in gestation week 20 and/or 21 can be selected from the group consisting of 60 ng/ml, 70 ng/ml, 80 ng/ml, 90 ng/ml, 95 ng/ml, 100 ng/ml, 105 ng/ml, 110 ng/ml, 115 ng/ml, 120 ng/ml and 130 ng/ml or any interval limited by any two of said levels.

The standard level of PAPP-A2 protein in a plasma sample taken in gestation week 22 and/or 23 can be selected from the group consisting of 60 ng/ml, 70 ng/ml, 80 ng/ml, 90 ng/ml, 95 ng/ml, 100 ng/ml, 105 ng/ml, 110 ng/ml, 115 ng/ml, 120 ng/ml and 130 ng/ml or any interval limited by any two of said levels.

The standard level of PAPP-A2 protein in a plasma sample taken in gestation week 24 and/or 25 can be selected from the group consisting of 60 ng/ml, 70 ng/ml, 80 ng/ml, 100 ng/ml, 120 ng/ml, 140 ng/ml, 160 ng/ml, 180 ng/ml, 200 ng/ml, and 220 ng/ml or any interval limited by any two of said levels.

The standard level of PAPP-A2 protein in a plasma sample taken in gestation week 26 can be selected from the group consisting of 80 ng/ml, 100 ng/ml, 120 ng/ml, 140 ng/ml, 160 ng/ml, 180 ng/ml, 200 ng/ml, and 220 ng/ml or any interval limited by any two of said levels.

The standard level of PAPP-A2 protein in a plasma sample taken in gestation week 27 can be selected from the group consisting of 100 ng/ml, 120 ng/ml, 140 ng/ml, 160 ng/ml, 180 ng/ml, 200 ng/ml, 220 ng/ml, 240 ng/ml and 260 ng/ml or any interval limited by any two of said levels.

The standard level of PAPP-A2 protein in a plasma sample taken in gestation week 28 can be selected from the group consisting of 120 ng/ml, 140 ng/ml, 160 ng/ml, 180 ng/ml, 200 ng/ml, 220 ng/ml, 240 ng/ml, 250 ng/ml, 260 ng/ml and 280 ng/ml or any interval limited by any two of said levels.

The standard level of PAPP-A2 protein in a plasma sample taken in gestation week 29 can be selected from the group consisting of 120 ng/ml, 140 ng/ml, 160 ng/ml, 180 ng/ml, 200 ng/ml, 220 ng/ml, 240 ng/ml, 250 ng/ml, 260 ng/ml and 280 ng/ml or any interval limited by any two of said levels.

The standard level of PAPP-A2 protein in a plasma sample taken in gestation week 30 can be selected from the group consisting of 140 ng/ml, 160 ng/ml, 180 ng/ml, 200 ng/ml, 220 ng/ml, 240 ng/ml, 250 ng/ml, 260 ng/ml, 280 ng/ml and 300 ng/ml or any interval limited by any two of said levels.

The standard level of PAPP-A2 protein in a plasma sample taken in gestation week 31 can be selected from the group consisting of 150 ng/ml, 160 ng/ml, 180 ng/ml, 200 ng/ml, 220 ng/ml, 240 ng/ml, 260 ng/ml, 280 ng/ml, 300 ng/ml, 320 ng/ml and 340 ng/ml or any interval limited by any two of said levels.

The standard level of PAPP-A2 protein in a plasma sample taken in gestation week 32 can be selected from the group consisting of 220 ng/ml, 240 ng/ml, 260 ng/ml, 280 ng/ml, 300 ng/ml, 320 ng/ml, 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, and 600 ng/ml or any interval limited by any two of said levels.

The standard level of PAPP-A2 protein in a plasma sample taken in gestation week 33 can be selected from the group consisting of 260 ng/ml, 280 ng/ml, 300 ng/ml, 320 ng/ml, 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml and 650 ng/ml or any interval limited by any two of said levels.

The standard level of PAPP-A2 protein in a plasma sample taken in gestation week 34 can be selected from the group consisting of 260 ng/ml, 280 ng/ml, 300 ng/ml, 320 ng/ml, 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml and 650 ng/ml or any interval limited by any two of said levels.

The standard level of PAPP-A2 protein in a plasma sample taken in gestation week 35 can be selected from the group consisting of 300 ng/ml, 320 ng/ml, 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml and 750 ng/ml or any interval limited by any two of said levels.

The standard level of PAPP-A2 protein in a plasma sample taken in gestation week 36 can be selected from the group consisting of 320 ng/ml, 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml and 750 ng/ml or any interval limited by any two of said levels.

The standard level of PAPP-A2 protein in a plasma sample taken in gestation week 37 can be selected from the group consisting of 340 ng/ml, 350 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml and 750 ng/ml or any interval limited by any two of said levels.

The standard level of PAPP-A2 protein in a plasma sample taken in gestation week 38 can be selected from the group consisting of 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml and 750 ng/ml or any interval limited by any two of said levels.

The standard level of PAPP-A2 protein in a plasma sample taken in gestation week 39 can be selected from the group consisting of 220 ng/ml, 240 ng/ml, 260 ng/ml, 280 ng/ml, 300 ng/ml, 320 ng/ml, 340 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml, 750 ng/ml, 775 ng/ml and 800 ng/ml or any interval limited by any two of said levels.

The standard level of PAPP-A2 protein in a plasma sample taken in gestation week 40 can be selected from the group consisting of 340 ng/ml, 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml, 750 ng/ml, 775 ng/ml and 800 ng/ml, 825 ng/ml, 850 ng/ml, 875 ng/ml and 900 ng/ml or any interval limited by any two of said levels.

The standard level of PAPP-A2 protein in a plasma sample taken in gestation week 41 can be selected from the group consisting of 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml, 750 ng/ml, 775 ng/ml and 800 ng/ml, 825 ng/ml, 850 ng/ml, 875 ng/ml and 900 ng/ml or any interval limited by any two of said levels.

The standard level of PAPP-A2 protein in a plasma sample taken in gestation week 42 can be selected from the group consisting of 360 ng/ml, 380 ng/ml, 400 ng/ml, 425 ng/ml, 450 ng/ml, 475 ng/ml, 500 ng/ml, 525 ng/ml, 550 ng/ml, 575 ng/ml, 600 ng/ml, 625 ng/ml, 650 ng/ml, 675 ng/ml, 700 ng/ml, 725 ng/ml, 750 ng/ml, 775 ng/ml and 800 ng/ml, 825 ng/ml, 850 ng/ml, 875 ng/ml and 900 ng/ml or any interval limited by any two of said levels.

The standard level of PAPP-A2 protein or the normal level of PAPP-A2 in a certain gestation week in the plasma can be determined as the 90^{th}, 91^{th}, 92^{nd}, 93^{rd}, 94^{th}, 95^{th}, 96^{th}, 97^{th}, 98^{th} or 99^{th} centile of the level of PAPP-A2.

The standard level of PAPP-A2 protein or the normal level of PAPP-A2 in a certain gestation week 15, 16 or 17 can be around 47 ng/ml such as 47 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 18 can be around 46 ng/ml such as 46 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 19 can be around 52 ng/ml such as 52 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 20, 21, 22, 23, 24 or 25 can be around 56 ng/ml such as 56 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 26 can be around 62 ng/ml such as 62 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 27 can be around 77 ng/ml such as 77 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 28 can be around 93 ng/ml such as 93 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 29 can be around 97 ng/ml such as 97 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 30 can be around 109 ng/ml such as 109 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 31 can be around 118 ng/ml such as 118 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 32 can be around 178 ng/ml such as 178 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 33 or 34 can be around 192 ng/ml such as 192 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 35, 36 or 37 can be around 260 ng/ml such as 260 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 38 can be around 278 ng/ml such as 278 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 39 can be around 149 ng/ml such as 149 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 40 can be around 248 ng/ml such as 248 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 41 can be around 264 ng/ml such as 264 ng/ml ±20%, ±18%, ±16%, ±14%, ±12%, ±10%, ±8%, ±6%, ±4%, ±2%, or ±1%, or any intervals limited by any two of said levels.

The standard level of PAPP-A2 protein or the normal level of PAPP-A2 in a certain gestation week 42 can be around 190 ng/ml such as 190 ng/ml ±20%, ±18%, ±16%, ±14%, ±12%, ±10%, ±8%, ±6%, ±4%, ±2%, or ±1%, or any intervals limited by any two of said levels.

When the level of PAPP-A2 is measured as an amount of PAPP-A2 protein, the PAPP-A2 protein is preferably measured by immunochemical analysis wherein PAPP-A2 protein is detected by at least one monoclonal antibody. PAPP-A2 protein may also be detected in a complex comprising at least one additional component, preferably a polypeptide complex. PAPP-A2 may also be detected as a PAPP-A2 monomer or as a PAPP-A2 dimer.

Further aspects of the disclosure relates to a method of diagnosing and/or prognosing preeclampsia or a related disease, said method comprising the steps of
i) performing a method for detecting PAPP-A2 or measuring the level of PAPP-A2, and
ii) performing a method for detecting one or more biomarkers for preeclampsia besides PAPP-A2 (i.e. one or more secondary markers for preeclampsia) or measuring the level of one or more biomarkers besides PAPP-A2 (i.e. one or more secondary markers for preeclampsia), and
iii) diagnosing and/or prognosing preeclampsia or a related disease.

In a yet further aspect of the disclosure there is provided a method for detecting expression of a PAPP-A2 polynucleotide in a biological sample, said method comprising the steps of
i) providing a biological sample putatively containing a PAPP-A2 polynucleotide and
ii) contacting the biological sample with a polynucleotide comprising a strand that is i) complementary to the polynucleotide and ii) capable of hybridizing thereto, and
iii) allowing hybridization to occur, and
iv) detecting the hybridization complex obtained in step iii),
wherein the presence of the hybridization complex is indicative of the expression in the biological sample of the PAPP-A2 polynucleotide or a fragment thereof.

### Types of diseases

The method can be used for assessing and predicting preeclampsia.

In one embodiment the preeclampsia can be in the first, second, and/or third trimester of pregnancy.
More specifically the disease can be early preeclampsia, late preeclampsia, mild preeclampsia, severe preeclampsia and superimposed preeclampsia.

### Detection reagent

In one embodiment PAPP-A2 is detected by PAPP-A2 specific antibodies. Recombinant PAPP-A2 can be used to generate antibodies. Monospecific antibodies to PAPP-A2 can e.g. be purified from mammalian antisera containing antibodies reactive against PAPP-A2 or can be prepared as monoclonal antibodies reactive with PAPP-A2 using standard techniques.

Monospecific antibody as used herein is defined as a single antibody species or multiple antibody species with homogenous binding characteristics for PAPP-A2. Homogenous binding as used herein refers to the ability of the antibody species to bind to a specific antigen or epitope, such as those associated with the PAPP-A2, as described above. PAPP-A2 specific antibodies can be raised by immunizing animals such as mice, rats, guinea pigs, rabbits, goats, horses and the like, with rabbits or mice being preferred, with an appropriate concentration of PAPP-A2 either with or without an immune adjuvant.

Polyclonal and/or monoclonal antibodies against PAPP-A2 can be constructed for measurement of PAPP-A2 antigen in body fluids or tissue and cell extracts.

Any PAPP-A2 detection reagents described in the prior art can be used for detection of PAPP-A2.

### Assay

The assays for detection of PAPP-A2 include, but are not limited to, precipitation, passive agglutination, enzyme-linked immunosorbent assay (ELISA) techniques, and radioimmunoassay (RIA) techniques. Any quantitative or semi-quantitative detection method can be used for determination of the PAPP-A2 level.

For example, in one such ELISA, a sandwich assay can be constructed where antigen present in an sample is caught by immobilized polyclonal anti(PAPP-A2). Detection is then performed by the use of one or more monoclonal PAPP-A2 antibodies and peroxidase conjugated anti(murine IgG). In another assay, antigen present in an sample is caught by immobilized polyclonal anti(PAPP-A2), and detected using biotinylated polyclonal anti(PAPP-A2).

The PAPP-A2 antibodies can be used either for a qualitative or a quantitative or a semi-quantitative determination of PAPP-A2.

Native/mature PAPP-A2 from sources such as human plasma or serum, tissue extracts such as placenta extracts can be analysed.

Using polyclonal or monoclonal antibodies against PAPP-A2 a number of assays may be constructed for measurement of PAPP-A2 antigen in body fluids or tissue and cell extracts. Kits based on antibodies may be used for diagnostic purposes. The assays include, but are not limited to, precipitation, passive agglutination, enzyme-linked immunosorbent assay (ELISA) techniques, and radioimmunoassay (RIA) techniques.

For example, in one such ELISA, a sandwich assay can be constructed where antigen present in an sample is caught by immobilized polyclonal anti(PAPP-A2). Detection is then performed by the use of one or more monoclonal PAPP-A2 antibodies and peroxidase conjugated anti(murine IgG). In another assay, antigen present in an sample is caught by immobilized polyclonal anti(PAPP-A2), and detected using biotinylated polyclonal anti(PAPP-A2). Assays can be calibrated using purified PAPP-A2 to construct a standard curve by serial dilution. The concentration of PAPP-A2 in solution in a purified form can be accurately measured by amino acid analysis (Sottrup-Jensen, 1993, Biochem Mol Biol Int 30, 789-94).

Another way of analysing the PAPP-A2 protein level is by use mass spectrometry based methods. Mass spectrometry is not inherently. The intensity of a peak in a mass spectrum is not a good indicator of the amount of the analyte in the sample, although differences in peak intensity of the *same* analyte between multiple samples accurately reflect relative differences in its abundance. One approach for relative quantitation is to separately analyze samples by MS and compare the spectra to determine peptide abundance in one sample relative to another, as in label-free quantitation strategies. An approach for relative quantitation of PAPP-A2 that is more costly and time-consuming, though less sensitive to experimental bias than label-free quantitation, entails labeling the samples with stable isotope labels that allow the mass spectrometer to distinguish between PAPP-A2 in separate samples. One type of label, isotopic tags, consist of stable isotopes incorporated into protein crosslinkers that causes a known mass shift of the labeled protein or peptide in the mass spectrum. Differentially labeled samples are combined and analyzed together, and the differences in the peak intensities of the isotope pairs accurately reflect difference in the abundance of the corresponding proteins. Absolute proteomic quantitation using isotopic peptides entails spiking known concentrations of synthetic, heavy isotopologues of target peptides into an experimental sample and then performing LC-MS/MS. As with relative quantitation using isotopic labels, peptides of equal chemistry co-elute and are analyzed by MS simultaneously. Unlike relative quantitation, though, the abundance of the PAPP-A2 target peptide in the experimental sample is compared to that of the heavy peptide and back-calculated to the initial concentration of the standard using a pre-determined standard curve to yield the absolute quantitation of the target peptide. Relative quantitation methods include: Isotope-coded affinity tags (ICAT); Isobaric labeling, Tandem mass tags (TMT), Isobaric tags for relative and absolute quantitation (iTRAQ), Label-free quantification, Metal-coded tags (MeCATs), N-terminal labelling and Stable isotope labeling with amino acids in cell culture (SILAC). Absolute quantitation can be performed using Selected Reaction Monitoring (SRM).

In a yet further aspect of the invention there is provided a method for detecting expression of a PAPP-A2 polynucleotide according to the invention in a biological sample. The biological sample is contacted with a polynucleotide comprising a strand that is i) complementary to the polynucleotide according to the invention and ii) capable of hybridizing thereto. Hybridization is allowed to occur, and the hybridization complex is detected by any method disclosed in the prior art.

### Measurement of PAPP-A2 activity

PAPP-A2 contains conserved amino acid stretches that classify it as a putative metalloproteinase of the metzincin superfamily (Stocker et al., 1995, Protein Sci 4, 823-40). It has been experimentally verified that PAPP-A2 does exhibit proteolytic activity by demonstrating its cleavage of insulin-like growth factor binding protein (IGFBP)-5 (US7,083,940).

In general, proteolytic activity of PAPP-A2 against potential protein substrates may be evaluated by the incubation of purified or partially purified PAPP-A2 with the potential substrate under a variety of experimental conditions (such as for example temperature, buffer composition, ionic strength, and pH). Enzymatic activity of PAPP-A2 against the protein in question can be evaluated by SDS-PAGE (in which degradation or release of well defined proteolytic fragment(s) will be evident), or by high-pressure liquid chromatographic detection of released peptide(s). By means of such procedures, other substrate targets of PAPP-A2 may be identified. Incubation with a variant of PAPP-A2 where, for example, a residue in the active site has been substituted to obtain an inactive enzyme, serves as a proper negative control.

Random peptide libraries consisting of all possible combinations of amino acids attached to a solid phase support may be used to identify peptides that can be cleaved by PAPP-A2. Identification of such peptides may be accomplished by screening a peptide library with recombinant soluble PAPP-A2. Methods for expression and purification of the enzyme are described above and may be used to express recombinant full length PAPP-A2 or fragments, analogs, or derivatives thereof depending on the functional domains of interest. For further details, see (Meldal, 1998, Methods Mol Biol 87, 65-74; Meldal, 1998, Methods Mol Biol 87, 51-7). Alternatively, peptide substrates may be derived from identified protein substrates of PAPP-A2.

Alternatively, phage display of peptide libraries may be used to identify peptides that can be cleaved by PAPP-A2 (Matthews and Wells, 1993, Science 260, 1113-7).

Peptides that function as PAPP-A2 substrates may function in assays for the detection of PAPP-A2 proteolytic activity in body fluids or tissue and cell extracts. Substrate peptides may be derivatized to function in an assay based on quenched-fluorescence (Meldal, 1998, Methods Mol Biol 87, 65-74). Kits based on such, or other, techniques may be used for diagnostic purposes in pathologies where measurement of PAPP-A2 activity is relevant.

The activity of PAPP-A2 can be used as a marker for preeclampsia.

### Samples

PAPP-A2 can be measured in any type of sample from a pregnant woman including body fluid samples and cell or tissue samples. The body fluid sample may be any useful body fluid sample, such as a blood sample including a blood sample, a plasma sample, a serum sample, a urine sample, a saliva sample, a folliculart fluid sample, a cerebrospinal fluid sample, or an amniotic fluid sample.

In one embodiment the biological sample is preferably selected from the group consisting of blood, urine, pleural fluid, oral washings, tissue biopsies, and follicular fluid.

Tissue samples can derive from any tissue or a homogenate thereof. A preferred tissue sample is a placenta tissue sample e.g. obtained by a placenta biopsy.

### Sampling time during gestation

The sample to be analysed can be obtained from the subject at any time during pregnancy such as in gestation week 17, 18, 19, 20, 21, 22, 23, 24, 25, 26, 27, 28, 29, 30, 31, 32, 33, 34, 35, 36, 37, 38, 39, 40, 41 and/or 42.

One or more samples from the same subject can be analysed such as 2, 3, 4, 5, 6, 7, 8, 9, 10 or more than 10 samples. The more than one sample can be obtained in the same gestation week or in different gestation weeks.

The first analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

The second analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

The third analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

The fourth analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

The fifth analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

The sixth or any further analysis of the PAPP-A2 level and/or the one or more secondary markers can be performed on a sample derived from gestation week 17, 18, 19, 20, 21, 22, 23, 24, 25, 26, 27, 28, 29, 30, 31, 32, 33, 34, 35, 36, 37, 38, 39, 40, 41 and/or 42.

In one embodiment more than one analysis is performed if the first or a subsequent analysis was inconclusive with respect to if the subject has preeclampsia or has an increased risk of developing preeclampsia.

In one embodiment more than one analysis is performed if the first or a subsequent analysis showed that the subject has an increased risk of developing preeclampsia. If a subject is having a risk of developing preeclampsia.

The gestation time of the subject and the gestation time of the population used to determine the standard value are within three weeks of each other, within two weeks of each other, or within one week of each other.

### Detection of "free" PAPP-A2 and "complexed" PAPP-A2

The present invention also relates to a method for detection of specific complexes in which PAPP-A2 is present, and/or to the detection of both "free" PAPP-A2 and "complexed" PAPP-A2, and/or to detection of only "free" PAPP-A2.

The complexes PAPP-A-ProMBP and/or ProMBP-AGT could also be used as one or more secondary markers.

### Combination of diagnostic and/or prognostic methods

In one embodiment the present invention relates to methods of assessing, predicting and diagnosing preeclampsia as well wherein the level of PAPP-A2 and one or more further biomarkers (secondary markers) associated with preeclampsia is detected or monitored. The number of secondary markers associated with preeclampsia can be 1, 2, 3, 4, 5, 6, 7, 8, 9, 10 or more than 10 different secondary markers.

The level of the one or more secondary markers can be measured at the same gestational age as the level of PAPP-A2 is measured or alternatively the level of the secondary marker can be determined at a different gestational age than where the PAPP-A2 level is determined.

The level of the one or more secondary markers can be measured in the same diagnostic assay as where the level of PAPP-A2 is measured or alternatively the level of the secondary marker can be determined in a different diagnostic assay than where the PAPP-A2 level is determined.

The level of the one or more secondary markers can be measured in the same type of sample - such as e.g. a serum, a blood sample, a placenta biopsy sample or a urine sample - as the sample wherein the level of PAPP-A2 is measured or alternatively the level of the secondary marker can be determined in a different type of sample than the sample wherein the PAPP-A2 level is determined.

In on embodiment the one or more secondary markers associated with preeclampsia can be one or more secondary markers disclosed in one or more of the US patent applications selected from the group consisting of US 20120189632, US 20120142559, US20120135427, US 20120101021, US 20120040371, US 20110318809, US20110280863, US 20110269136, US20110171650, US 20110104107, US20110059904, US 20100323352, US 20100291612, US20100227342, US20100190181, US 20100113286, US 20100105070, US 20100017143, US20100016173, US 20080233583 and US 20060252068.

The content of US 20120189632, US 20120142559, US 20120135427, US 20120101021, US 20120040371, US 20110318809, US 20110280863, US 20110269136, US20110171650, US 20110104107, US 20110059904, US 20100323352, US 20100291612, US20100227342, US 20100190181, US 20100113286, US 20100105070, US 20100017143, US20100016173, US 20080233583 and US 20060252068 is incorporated in in their entireties herein.

The one or more secondary markers can e.g. be activin A, PLGF, sEndoglin, PAPP-A, PAPP-A-ProMBP and/or ProMBP-AGT. Preferred secondary markers are activin A, sEndoglin, PAPP-A, PAPP-A-ProMBP, and ProMBP-AGT. More preferred secondary markers are activin A and sEndoglin. A preferred combination of secondary markers is activin A and sEndoglin.

In another embodiment the one or more secondary markers associated with preeclampsia can be one or more secondary markers selected from the group consisting of insulin-like growth factor-binding protein complex acid labile subunit (IGFALS), SEPP1, s-Endoglin (ENG or s-ENG), quiescin Q6 (QSOX1), peroxiredoxin-2 (PRDX2), blood glucose level, body mass index (BMI), 'high density lipoprotein level', 'ratio of total cholesterol to high density lipoprotein', metabolic syndrome, triglycerides level, vascular endothelial growth factor receptor 3 (FLT4), lysosomal Pro-X carboxypeptidase (PRCP), peroxiredoxin-1 (PRDX1), leucyl-cystinyl aminopeptidase (LNPEP), tenascin-X (TNXB), basement membrane-specific heparan sulfate proteoglycan core protein (HSPG2), cell surface glycoprotein (CD146, MUC18, MCAM), phosphatidylinositol-glycan-specific phospholipase D (GPLD1), collagen alpha-3(VI) chain (COL6A3), Kunitz-type protease inhibitor 1 (SPINT1), hepatocyte growth factor-like protein (MST1), probable G-protein coupled receptor 126 (GPR126), intercellular adhesion molecule 3 (ICAM3), C-reactive protein (CRP), disintegrin and metalloproteinase domain-containing protein 12 (ADAM12), phosphatidylcholine-sterol acyltransferase (LCAT), roundabout homolog 4 (ROBO4), ectonucleotide pyrophosphatase/phosphodiesterase family member 2 (ENPP2), protein S100-A9 (S100A9), SEPP1, ENG, QSOX1, PRDX2, PIGF, Activin A, P-Selectin, angiogenic factors, VEGF, PIGF, sFIt-1, Chaperonin, ER-60 protease, Alpha-enolase, Isocitrate dehydrogenase I, Aldehyde reductase, Chain B Fidarestat Bonded to human Aldose reductase, Voltage-dependent anion channel 1, Nuclear chloride channel, Chain H Cathepsin D, Phosphoglycerate mutase I, Endoplasmatic reticulum protein, PSMA2 protein, Glutathione S-transferase, Ig heavy chain v region, Smooth muscle myosin alkali light chain, Fatty acid binding protein, supramolecular aggregate of misfolded proteins that is associated with (is a causative factor in the pathology of) preeclampsia in e.g. a urine sample or placenta tissue sample, supramolecular aggregate of misfolded proteins comprising serpina-1 (alpha-1 antitrypsin) or a fragment of serpina-1, supramolecular aggregate further comprising at least one of ceruloplasmin, heavy-chain IgG, light-chain IgG and interferon inducible protein 6-16 (IFI6), placental chondroitin 4-O-sulfotransferase 1 (C4ST), chondroitin 6-sulfotransferase (C6S), heparan sulfate 6-O-sulfotransferase 1 (HS6S), dermatan/chondroitin sulfate 2-sulfotransferase (CS-2OST), uronic acid-2-sulfate (UA2S), glycosaminoglycan (GAG) synthesis regulatory enzyme from a placental tissue, ABLIM2, ACACA, ACOT8, ASCL2, AHSG, ALDH1A2, ALS2CL, ANXA13, APC, AQP2, ARFGEF2, ART1, ASCL2, ATP7B, AXIN1, BDKRB2, BICD1, C3, C4BPA, C4orf10, C6orf142, CCK, CD52, CDH15, CENTG3, CFHR1, CHERP, CHF, CHRDL1, CHST6, CFHR1, CLASP2, CLCN7, CLDN6, CTAG2, COL5A1, COL9A3, CPM, CRI, CRYBB1, CUL1, CYP4A11, CXCL9, DEPDC7, DHRS2, DLGAP1, DPYSL4, ELL2, EGLN3, EGR1, EPAS1, F11R, F2R, FKBP1A, FLRT2, FLT4, FN1, FOS, FOSB, FPRL2, FSTL3, HSP3, FUT6, FZD5, GATA1, GNG4, GNG7, GNLY, GRP109B, GTPBP2, GZMB, HBE1, HBZ, HCFC1, HEXA, HLA-DQA1, HOXB7, HPS3, HTR2B, HYDIN, IGFBP1, IGKC, IKZF1, IL15, IL1B, IL1RL1, IL2RB, ISG20, ITGB6, KCNH2, KCNIP3, KCNQ1, KISS1R, KLRC2, KRT14, LAIR2, LIPH, LOC440157, LRAP, LSS, LTBR, MAGEB6, MAOB, MAP2K7, MGA, MMD, MMP12, MSR1, MUC4, MUC15, MYL9, NDP, NDUFV2, NINJ2, NKX2-5, NMNAT3, NOG, NTN1, OPRL1, OXGR1, P4HA3, PACAP, PAEP, PDE4C, PDE9A, PITPNC1, PLAC8, PRDM1, PRG2, PRL, PSCD2, PTPRN, PTPRS, RAB12, RAD52, RBP4, REST, RHD, RORB, RUFY3, S100A8, S100A12, SART3, SCARA5, SDHAL1, SEMA3C, SERPINA3, SEZ6L, SLC13A4, SLC13A5, SLC2A3, SLC16A6, SLC04A1, SPDEF, SPG20, SPOCK1, SPRR2B, SRF, SSTR1, ST6GALNAC4, STXBP2, THBS4, TMC4, TNRC9, TPM1, TRA@, TRIM3, UGGT2B7, WNT10B, WT1, ZP3, methylation of Maspin gene (differentially methylated in fetal DNA and in maternal DNA), calcyclin, expression level of calcyclin in chorionic villi, levels of angiogenic factors, specifically VEGF, PIGF and sFlt-1 in urine samples, histidine and ketone bodies, fatty acid binding protein 4 (FABP4), Peroxiredoxin 6, enoyl-CoA hydratase (ECHS1), human olacental lactogen, DELTA3,5-DELTA2,4-dienoyl-CoA isomerase (ECH1), Per6, heat shock protein beta-1 (HSP27), stathmin, FABP4, ECHS1, ECH1, heat shock protein E-backward-1, lipocortin, prostaglandin dehydrogenase 1, proliferation-associated protein 2G4, placental growth hormone (chorionic sommatomammotropin hormone (CSH1), estradiol 17-beta dehydrogenase, macrophage capping protein, levels of free haemoglobin, particularly free fetal haemoglobin, endothelin, soluble forms-like tyrosine kinase-1 (sFIt-1), angiotensin-II, Alpha-1 B-glycoprotein, - Actin, Apolipoprotein B- 100, Apolipoprotein C-II, Apohpoprotein C-III, C4b-binding protein beta chain, Cathepsin D, Choriogonadotropin subunit beta, Cholinesterase, Chorionic somatomammotropin hormone, Cystatin-C, Endoglin, Coagulation factor XI, Coagulation factor VII, Fibronectin, Filamin-A, Heparin cofactor 2, Hepatocyte growth factor-like protein, Histidine-rich glycoprotein, Insulin-like growth factor-binding protein 2, Laminin subunit beta-1, Lipopolysaccharide-binding protein, Matrix metalloproteinase-9, Plastin-2, Profilin-1, Pregnancy-specific beta-1-glycoprotein, Receptor-type tyrosine-protein phosphatase gamma, Pregnancy zone protein, Plasma retinol-binding protein, SH3 domain-binding glutamic acid-rich-like protein 3, Transgehn-2, Talin-1, Tropomyosin alpha-4 chain, Vasorin, Vascular endothelial growth factor receptor 3, Vinculin, von Willebrand factor, Pappalysin-2, Alpha-2-antiplasmin, Actin, Afamin, Antithrombin-III, Apolipoprotein A-II, Attractin, Beta-2-microglobulin, Transforming growth factor-beta-induced protein ig-h3, C4b-binding protein alpha chain, Carboxypeptidase B2, Complement factor D, Cartilage acidic protein 1, Dopamine beta-hydroxylase, Coagulation factor XIII B chain, Fibrinogen alpha chain, Rho GDP-dissociation inhibitor 2, Platelet glycoprotein Ib alpha chain, Haptoglobin-related protein, Platelet basic protein, Tubulin beta-1 chain, Thymosin beta-4, Vascular cell adhesion protein 1, Zinc-alpha-2-glycoprotein, Alpha-2-macroglobulin, Pappalysin-1, C-reactive protein, Serum amyloid P-component, Complement factor H-related 5, Protein piccolo, Xaa-Pro dipeptidase, Protein bassoon, Dystroglycan, Catalase, Carbonic anhydrase 1, Intracellular adhesion molecule 1, Serotransferrin, Galectin-3-binding protein, Peroxiredoxin-2, Biphosphoglycerate mutase, Corticosteroid-binding globulin, Carbonic anhydrase 2, Adenomatous polyposis coli protein, Latent-transforming growth factor beta-binding, Coagulation factor IX, Hepatocyte growth factor activator, Complement C1q subcomponent subunit C, Complement C1q subcomponent subunit B, Cartilage oligomeric matrix protein, gamma-enteric smooth muscle, Mast/stem cell growth factor receptor, Platelet glycoprotein V, Roundabout homolog 4, Extracellular matrix protein 1, Complement C1q subcomponent subunit A, Phospholipid transfer protein, ADAMTS-13, Plasma protease C1 inhibitor, Apolipoprotein F, Noelin, Low affinity immunoglobulin gamma Fc region receptor, CD44 antigen, Macrophage mannose receptor 1, Fibrinogen beta chain, Membrane copper amine oxidase, Alpha-1-acid glycoprotein 1, Cadherin-5, Fructose-biphosphate aldolase A, Probable G-protein couple receptor 126, 14-3-3 protein zeta/delta, Cofilin-1, Glycealdehyde-3-phosphate dehydrogenase, N-acetylglucosamine-1-phosphotransferase subunit gamma, Alpha-actinin-1, Phosphoglycerate mutase 1, Term-like transcript 1 protein, Glutathione S-transferase P, Leucyl-cystinyl aminopeptidase, Adenylyl cyclase-associated protein 1, Peptidyl-prolyl cic-trans isomerase A, Transketolase, Phosphoglycerate kinase 1, Leptin, cortocotropin releasing hormone, inhibit Beta A, chorionic gonadotropin beta polypeptide, NA, Fms-related tyrosine kinase 1 (VEGFR), sialic acid binding Ig-like lectin 6, luteinizing hormone beta polypeptide, B-cell CLL/lymphoma 6, inhibin alpha, pappalysin 2, endoglin (Osler-Rendu-Weber syndrome 1), Sperm associated antigen 4, Retinol dehydrogenase 13 (all-trans and 9-cis), glucosidase beta acid, protease serine, 11 (IGF binding), SHS multiple domains 1, Solute carrier family 2, synapse defective 1, Rho GTPase, scavenger receptor class B member 1, solute carrier organic anion, mannosidase alpha class 1C member 1, frizzled homolog 10, KIAA1211 protein, UDP-Gal:betaGlcNAc, BTG family, member 2, hypothetical protein MGC17839, potassium channel subfamily K member 3, chromosome 1 open reading frame 139, glutathione S-transferase A3, fibrillin 2, carbonic anhydrase X, ankyrin repeat and SOCS box-containing 2, hydroxysteroid (17-beta), dehydrogenase 1, anthrax toxin receptor 1, LEP, CRH, LPL, INHBA, LPL, CRH, FLT1, CGB, CGB5, CGB7, FABP4, BCL6, INHBA, SIGLEC6, LHB, INHA, FSTL3, ADFP, TFPI, ERO1L, ENG, MME, CALM1, KIAA1102, LTF, RDH13, MBD2, SASH1, KIF2, PPL, NDRG1, SPAG4, KIAA1102, TPBG, MBD2, SLCO4A1, HA-1, LGALS8, BTNL9, EPS8L1, TFPI, C20orf38, CBLB, BCL6, HRASLS3, CALM1, SH3BP5, KIAA1984, SH3BP5, GBA, GBAP, EPS8L1, SH3MD1, EPS8L1, SASH1, HIG2, SLC6A8, FLJ43855, LRRC1, MAST4, FZD10, SLCO2A1, MTMR4, HSA9761, 7h3, PRSS11, PIK3AP1, GREM2, LGALS8, MAN1C1, SLC2A14, TUBA1, NEK11, TXNDC4, CSNK2A2, EFHD1, EBI3, KIF2, DUSP1, SCARB1, ADAM12, HEY1, LOC255743, SIPA1 L1, VDR, SASH1, PKD1L2, GLRX, DDR1, ARP3BETA, TIF1, LRRC1, FCN3, PHYHIP, TGOLN2, SREBF1, TTC17, RPL10, EVER1, ZC3HDC6, MGC17839, HCA112, MAPK8, KIAA1211, RASSF6, BTG2, FLJ90586, SLC26A2, MGC17839, FLJ23091, GSTA3, LEPREL1, FBN2, ASB2, CLDN1, ANTXR1, HSD17B1, TRIP10, IL1A, CGA, SYDE1, EPHA1, FLJ90575, B4GALT5, KCNK3, CA10, ADORA2B, MMP12, GKN1, C1orf139, IGFBP3, ABP1, FN1, INHBA, SLC21A2, SIGLEC6, KIAA0992, TIMP3, LEP and LPL.

The level of the one or more secondary markers can be determined at the protein or nucleotide level. The activity of the one or more secondary markers can also be determined.

The diagnosis of preeclampsia can further involve determination of one or more biophysical markers e.g. selected from uterine artery pulsatility index (PI) of the individual and mean arterial pressure (MAP) of the individual.

### Kit-of-parts

A kit-of-parts for diagnosing and/or prognosing preeclampsia comprises one or more reagents for determination of the PAPP-A2 level in a biological sample and instructions for use.

In one embodiment the kit-of-parts comprises one or more monoclonal or polyclonal antibodies specific for PAPP-A2.

### EXAMPLES

### Example 1

The protein level of PAPP-A2 was determined at different gestation weeks in a serum samples from women without preeclampsia and from women with preeclampsia.

### Procedure

All specimens and reagents were allowed to reach room temperature and mixed thoroughly by gentle inversion before use. Calibrators, controls, and unknowns were assayed in duplicate.

All serum samples reading higher than the highest calibrator were mixed thoroughly and diluted in the 0 ng/mL PAPP-A2 Cal A/Sample diluent prior to assay.

For pregnancy serum specimens: Dilute specimens 1:20 with the Cal A/Sample diluent before assay. Do not dilute Calibrators or Controls.

The following steps were performed:
1. Mark the microtitration strips to be used.
2. Pipette 50 µL of the Calibrator, Controls and Unknowns to the appropriate wells.
3. Add 50 µL of the PAPP-A2 Assay Buffer to each well using a semi-automatic dispenser.
4. Incubate the wells, shaking at a fast speed (600-800 rpm) on an orbital microplate shaker, for 1 hour at room temperature.
5. With 30-40 minutes remaining of incubation time, prepare the PAPP-A2 Antibody-Biotin Conjugate Solution by diluting the PAPP-A2 Biotin Conjugate Concentrate in PAPP-A2 Conjugate Diluent as described under the Preparation of the Reagents section of this insert.
6. Aspirate and wash each well 5 times for 30 seconds with Washing Solution using an automatic microplate washer.
7. Add 100 µL of the Antibody-Biotin Conjugate solution to each well using a semi-automatic dispenser.
8. Incubate the wells, shaking at a fast speed (600-800 rpm) on an orbital microplate shaker, for 1 hour at room temperature.
9. Aspirate and wash each well 5 times with the Wash Solution using an automatic microplate washer.
10. Add 100 µL of the Streptavidin-Enzyme Conjugate-RTU to each well using a semi-automatic dispenser.
11. Incubate the wells, shaking at a fast speed (600-800 rpm) on an orbital microplate shaker, for 30 minutes at room temperature.
12. Aspirate and wash each well 5 times with the Wash Solution using an automatic microplate washer.
13. Add 100 µL of the Substrate Solution to each well using a repeater pipette.
14. Incubate the wells, shaking at 600-800 rpm on an orbital microplate shaker for 4±1 minute at room temperature. Avoid exposure to direct sunlight.
15. Read the light output of the solution in the wells within 10 minutes, using a microplate luminometer.

**Typical Calibration Curve.**

| **Well Number** | **Well Contents** | **Mean RLU** | **Conc (ng/mL)** |
|---|---|---|---|
| **A1, A2** | Calibrators A | 1.79 (Blank) | 0 |
| **B1, B2** | B | 3.30 | 0.25 |
| **C1, C2** | C | 6.03 | 0.75 |
| **D1, D2** | D | 20.48 | 3.0 |
| **E1, E2** | E | 66.51 | 10.0 |
| **F1, F2** | F | 159.20 | 25.0 |

The results of the above experiments are described herein below.

Figure 2 shows PAPP-A2 levels from a variety of samples derived from pregnant woman without preeclampsia. Note the gradual increase of PAPP-A2 levels during pregnancy, peaking around 38 gestational weeks. Unlike other biomarkers for preeclampsia (e.g., activin A), normal PAPP-A2 levels during pregnancy gradually increase, rather than sharply increase late in the pregnancy term.

Figure 3 shows PAPP-A2 Levels in Normal and Preeclampsia Samples at the Same Average Gestational Age. Note at the 1^{st} Quartile, Normal PAPP-A2 levels are at approximately 50 ng/mL, which corresponds to a gestational week of 19 weeks (See Figure 2, which correlates PAPP-A2 levels to gestational age). There is a 1.4-fold difference between the values of PAPP-A2 in preeclamptic and normal samples. Note at the 3^{rd} Quartile, Normal PAPP-A2 levels are at approximately 143 ng/mL, which corresponds to a gestational week of 32 weeks. Now there is a 4-fold difference between the values of PAPP-A2 in preeclamptic and normal samples. This data demonstrates that while PAPP-A2 is a good marker for preeclampsia throughout pregnancy, the difference in PAPP-A2 values increases exponentially toward the end of gestation in women with preeclampsia. This trend is unlike PAPP-A2 levels in normal women, which gradually increase throughout pregnancy.

The gestational ages reported for all of the control (non PE) samples was analysed. In general, nearly all fell into one of four categories centred at 19, 29, 32 and 39 weeks' gestation. Any samples falling in the 'dead zone' before or between these categories were removed from the analysis. Figure 4 shows the remaining observations in controls, all indicated by open circles. The data are best fitted to a log distribution. If the lines are ignored it is clear that the values increase with gestational age and that the population is more variable (larger log SD) as gestational age increases.

The mean PAPP-A2 was regressed for each of the four groups versus the mean GA in each of the groups (Figure 5). They fit a straight line well. The same was done for the log SD (Figure 6) which also fit a straight line well. These two equations were then used to draw the reference lines on Figure 4 (labeled the 5^{th}, 50^{th} and 95^{th} centiles).

The advantages of fitting the data to distributions is that it allows reference data across the entire gestational age range from 18 weeks to term, even though the data are not present at certain gestational ages. Using these expected medians, all PAPP-A2 results can be converted to multiples of the median (MoM). This not only includes the controls displayed in Figure 4, but the cases of PE as well. A given MoM at one gestational age is not equivalent to that same MoM at a different gestational age. That is, they would represent different centiles of the normal distribution. Alternative the data can be represented as z-scores. In that type of analysis, a z-score at any gestational age indicates the same centile.

After reference ranges were established, the PAPP-A2 results in women who later develop preeclampsia were evaluated (Figure 8). These have been stratified by whether or not a sample was obtained at 34 weeks or later. If a later sample was obtained, then it is unlikely that the patient developed severe preeclampsia. These are indicated by triangles. The remaining observations from cases, that could potentially be more severe, are indicated by squares. It is clear that neither set of cases can be easily identified at the early gestational ages, while the separation increases as gestational age increases. More than half of the observations are above the 95^{th} centile of normal after 30 weeks gestation.

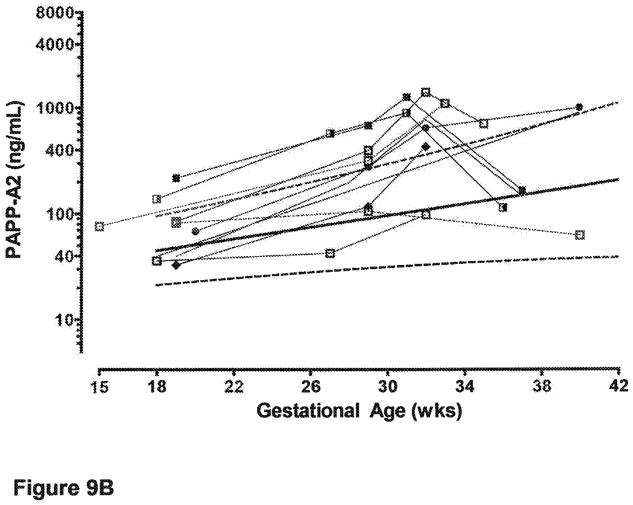

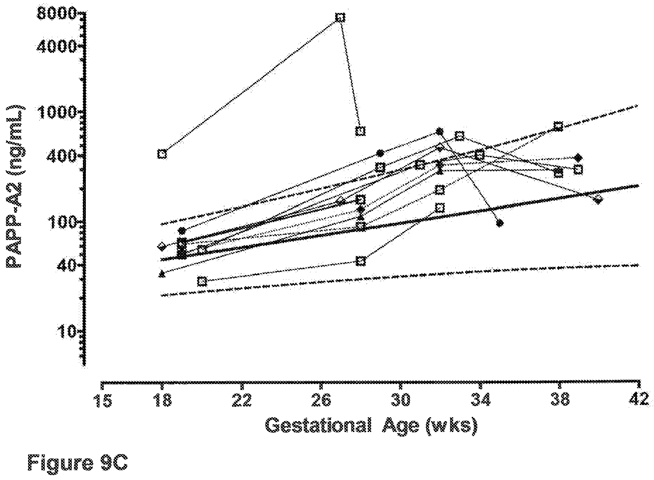

Figure 9 shows the patient-linked PAPP-A2 results for all women who developed preeclampsia. The 28 women are shown in three separate graphics so the patterns are more visible. Several have the interesting pattern of growing higher, then dropping steeply. Perhaps the PAPP-A2 is high for impending PE, but drops near the event.

### Example 2

### Determination of PAPP-A2 activity by cleavage of insulin-like growth factor binding protein (IGFBP)-5

Ligand blotting (Conover et al., 1993, J Clin Invest 91, 1129-37) with radiolabeled IGF-II (Bachem) was used to assay for activity against IGFBP-1 (from HepG2 conditioned medium), rIGFBP-2 (GroPep), rIGFBP-3 (gift of D. Powell), rIGFBP-4 (Austral), rIGFBP-5 (gift of D. Andress), and rIGFBP-6 (Austral). Of the six binding proteins, IGFBP-5 showed complete cleavage (Figure 10). IGFBP-3 was partially degraded (Figure 10). This cleavage was independent of the presence of IGF. Experiments were carried out with media from cells transfected with pPA2 or empty vector.

Figure 10 shows the activity of PAPP-A2 against IGFBP-1-6. Medium from 293T cells transfected with empty vector (-), or cDNA encoding PAPP-A2 (pPA2) (+) was incubated with each of the six IGFBPs (BP1-BP6), and the activity was assessed by ligand blotting using radiolabeled IGF-II. Complete cleavage of IGFBP-5 is evident from the absence of a signal in the BP5+ lane. Partial degradation of IGFBP-3 is also evident.

Accordingly IGFBP-5 can be used for determination of PAPP-A2 activity.

For further analysis, recombinant IGFBP-5 was produced in mammalian cells. In brief, human placental oligo-dT primed cDNA (Overgaard et al., 1999, Biol Reprod 61, 1083-9) was used as a template to amplify cDNA encoding human IGFBP-5 (Accession number M65062). Specific primers containing an *Xhol* site (5'-TCCGCTCGAGATGGTGTTGCTCACCGCGGT-3') and a *HindIII* site (5'-CGATAAGCTTCTCAACGTTGCTGCTGTCG-3') were used, and the resulting PCR product was digested and cloned into the *XhoI*/*HindIII* sites of pcDNA3.1/*Myc*-His(-)A (Invitrogen). The construct encoded the full-length proIGFBP-5, immediately followed by residues KLGP, the *myc* epitope (EQKLISEEDL), residues NSAVD, and six H-residues (amino acids are given as one letter code). The construct was verified by sequence analysis. Plasmid DNA for transfection was prepared by QIAprep Spin Kit (Qiagen). Cell culture and expression of recombinant IGFBP-5 was performed as described US7,083,940.

Cleavage analysis was performed by Western blotting (Figure 11). Briefly, recombinant IGFBP-5 as contained in 5 microL cell culture medium was incubated with culture supernatants (10 microL) from cells transfected with pPA2, pPA2-KO, or empty expression expression vectors (see US7,083,940). Phosphate buffered saline was added to a final volume of 50 microL. After incubation at 37 degrees Celsius for 12 hours, 15 microL of the reaction mixture was separated by reducing 16% SDS-PAGE, blotted onto a PVDF membrane, and the C-terminal cleavage product was detected with monoclonal anti-*c-myc* (clone 9E19, ATTC) using peroxidase-conjugated secondary antibodies (P260, DAKO), and enhanced chemiluminescence (ECL, Amersham).

### Example 3

### Samples

Blood samples were collected longitudinally during pregnancy from a cohort of pregnant women including women with pre-eclampsia (in mild or severe form) and normotensive pregnant women.

Samples were collected from the 18th week of pregnancy until delivery and divided into the following groups 1) early second trimester (i.e. approximately week 18 to 20), 2) early third trimester (i.e. approximately week 27 to 33 gestation) and 3) near term (i.e. approximately 38 to 40 weeks gestation).

Women scheduled to delivery at Randers Regional Hospital in Denmark were asked to participate in the project, and blood samples were collected during their pregnancy.

Women with pre-eclampsia, defined as de novo hypertension >140/90mmHg after the 20th week of pregnancy combined with proteinuria >300mg/L or ++ on a dipstick were identified. Both were measured on two occasions at least four hours apart.

Study group characteristics are shown in the Table herein below. The study conformed to the Declaration of Helsinki and was approved by the local ethical committee, and all women gave written informed consent.

**p-Values determined by Student's t-test**

| | **Pre-eclampsia, *n* = 32 Mean (SD)** | **Healthy pregnancy, *n* = 67 Mean (SD)** | ***p*-Value** |
|---|---|---|---|
| Age (years) | 32.8 (3.41) | 32.2 (4.2) | 0.39 |
| Blood pressure (mmHg) | 157/99 (13.8/5.7) | 111/69 (12.5/7.1) | <0.0001* |
| Urinary albumin (g/L) | 2.11 (4.3) | 0.014 g/l (0.0034) | <0.0001* |
| Gestational age at delivery (weeks) | 37.1 | 39.6 | <0.0001* |

| | | | |
|---|---|---|---|
| **p*<0.05. | | | |

Blood samples were collected from the 18th-19th week of pregnancy until delivery. Each woman provided a maximum of four blood samples.

### Analysis of PAPP-A2

The PAPP-A2 level in the blood samples were determined by PAPP-A2 ELISA (AnshLabs AL-109-i) as described herein below.

### Principle of PAPP-A2 ELISA

The PAPP-A2 ELISA is a quantitative three-step sandwich type immunoassay. In the first step Calibrators, Controls and unknown samples are added to anti-PAPP-A2 antibody coated micro titer wells and incubated. After first incubation and washing step, the wells are incubated with biotin labeled antibody conjugate. After a second incubation and washing step, the wells are incubated with streptavidin horseradish peroxidase conjugate (SHRP) solution. After the third incubation and washing step, the wells are incubated with substrate solution (TMB). After TMB incubation, an acidic stopping solution is added. In principle, the antibody-biotin conjugate binds to the solid phase antibody-antigen complex which in turn binds to the streptavidin enzyme conjugate. The antibody-antigen-biotin conjugate-SHRP complex bound to the well is detected by enzyme-substrate reaction. The degree of enzymatic turnover of the substrate is determined by dual wavelength absorbance measurement at 450 nm as primary test filter and 630 nm as reference filter. The absorbance measured is directly proportional to the concentration of PAPP-A2 in the samples and calibrators.

### Materials

### CAL-109A PAPP-A2 Calibrator A/Sample Diluent

One bottle, 10 mL, labeled PAPP-A2 Cal A/Sample Diluent, containing 0 ng/mL PAPP-A2 in a protein-based buffer with a non-mercury preservative. Store unopened at 2-8°C.

### CAL-109B -CAL-109F PAPP-A2 Calibrators B thru F

Five vials, labeled B-F containing a concentration 0.1, 0.3, 1.2, 4.0 and 10 ng/mL PAPP-A2 in protein based buffer with non-mercury preservative. Store at - 20°C. The PAPP-A2 concentration in the PAPP-A2 calibrators is traceable to manufacturer's working calibrators.

### CTR-109-I and CTR109-II PAPP-A2 Controls I & II

Two vials, labeled Levels I and II containing low and high PAPP-A 2 in protein based buffer with non-mercury preservative. Controls are shipped ambient. Store at - 20°C.

### PLT-109 Anti-PAPP-A2 Antibody Coated Microtitration Strips

One stripholder, containing 96 microtitration wells with PAPP-A2 antibody immobilized to the inside wall of each well. Store at 2-8°C in the resealable pouch with a desiccant to protect from moisture.

### ASB-109 PAPP-A2 Assay Buffer

One bottle, 8 mL, containing a protein-based (BSA)-buffer with a nonmercury preservative. Store at 2-8°C.

### CND-109 PAPP-A2 Biotin Conjugate Diluent

One bottle, 12 mL, containing a protein based buffer with a non-mercury preservative. Store at 2-8°C.

### BCC-109 PAPP-A2 Biotin Conjugate Concentrate

One vial, 0.4 mL containing a solution of anti-PAPP-A2 antibody biotin concentrate in a protein-based buffer with a non-mercury preservative. Dilute prior to use in PAPP-A2 Conjugate diluent. Store at 2-8°C. The dilution of this reagent should be made 15-30 minutes prior to use in the assay.

### SAR-109 PAPP-A2 Streptavidin-Enzyme Conjugate-Ready-to-Use (RTU)

One amber bottle, 12 mL, containing streptavidin-HRP (horseradish peroxidase) in a protein-based buffer and a non-mercury preservative. Store undiluted at 2-8°C.

### TMB-100 TMB Chromogen Solution

One bottle, 11 mL, containing a solution of tetramethylbenzidine (TMB) in buffer with hydrogen peroxide. Store at 2-8°C.

### STP-100 Stopping Solution

One bottle, 11 mL, containing 0.2 M sulfuric acid. Store at 2 to 30°C.

### WSH-100 Wash Concentrate A

One bottle, 60 mL, containing buffered saline with a nonionic detergent. Store at 2-30°C until expiration date. Dilute 25-fold with deionized water prior to use.

Other materials required:
1. Microtitration plate reader capable of absorbance measurement at 450 nm, 405nm and 630 nm.
2. Microtitration orbital plate shaker.
3. Microtitration plate washer.
4. Semi-automated/manual precision pipette to deliver 10-250 µL.
5. Vortex mixer.
6. Deionized water.
7. Disposable 12 x 75 mm culture tubes.
8. Tight fitting 12 x 75 mm tube racks.

### Protocol

Handling, processing and storing blood samples is described herein below:
- Allow samples to clot for two hours at room temperature or overnight at 4°C. Keep tubes stoppered at all times. Within two hours after centrifugation, transfer at least 500 µL of cell free sample to a storage tube. Tightly stopper the tube immediately.
- Samples if used within 24 hours may be stored at 4°C; otherwise samples must be stored at -20°C or -80°C to avoid loss of bioactivity and contamination.
- Remove residual fibrin and cellular matter prior to analysis.
- Avoid assaying lipemic, hemolyzed or icteric samples
- Avoid repeated freezing and thawing of samples. Thaw samples no more than 3 times.

### Preparation of Reagents

1. Wash Solution: Dilute wash concentrate 25-fold with deionized water.
2. PAPP-A2 Antibody-Biotin Conjugate Solution: The PAPP-A2 Antibody-Biotin Conjugate Concentrate should be diluted at a ratio of 1 part conjugate to 50 parts of PAPP-A2 Conjugate Diluent, according to the number of wells used.
3. Microtitration Wells: Select the number of coated wells required for the assay. The remaining unused wells should be placed in the resealable pouch with a desiccant. The pouch must be resealed to protect from moisture.

Allow all specimens and reagents to reach room temperature and mix thoroughly by gentle inversion before use. Calibrators, controls, and unknowns should be assayed in duplicate. All serum samples reading higher than the highest calibrator should be thoroughly mixed and diluted in the 0 ng/mL PAPP-A2 Cal A/Sample diluent prior to assay.

For pregnancy serum specimens: Dilute specimens 1:20 with the Cal A/Sample diluent before assay. Do not dilute Calibrators or Controls.
1. Mark the microtitration strips to be used.
2. Pipette 50 µL of the Calibrator, Controls and Unknowns to the appropriate wells.
3. Add 50 µL of the PAPP-A2 Assay Buffer to each well using a semiautomatic dispenser.
4. Incubate the wells, shaking at a fast speed (600-800 rpm) on an orbital microplate shaker, for 1 hour at room temperature.
5. With 30-40 minutes remaining of incubation time, prepare the PAPPA2 Antibody-Biotin Conjugate Solution by diluting the PAPP-A2 Biotin Conjugate Concentrate in PAPP-A2 Conjugate Diluent as described under the Preparation of the Reagents section of this insert.
6. Aspirate and wash each well 5 times for 30 seconds with Washing Solution using an automatic microplate
7. Add 100 µL of the Antibody-Biotin Conjugate solution to each well using a semi-automatic dispenser.
8. Incubate the wells, shaking at a fast speed (600-800 rpm) on an orbital microplate shaker, for 1 hour at room temperature.
9. Aspirate and wash each well 5 times with the Wash Solution using an automatic microplate washer.
10. Add 100 µL of the Streptavidin-Enzyme Conjugate-RTU to each well using a semi-automatic dispenser.
11. Incubate the wells, shaking at a fast speed (600-800 rpm) on an orbital microplate shaker, for 30 minutes at room temperature.
12. Aspirate and wash each well 5 times with the Wash Solution using an automatic microplate washer.
13. Add 100 µL of the TMB chromogen solution to each well using a precision pipette. Avoid exposure to direct sunlight.
14. Incubate the wells, shaking at 600-800 rpm on an orbital microplate shaker, for 10-12 min at room temperature.
15. Add 100 µL of the stopping solution to each well using a precision pipette.
16. Read the absorbance of the solution in the wells within 20 minutes, using a microplate reader set to 450 nm.

While reading the absorbance of the microtitration well, it is necessary to program the zero calibrator as a "Blank".

### Statistical analysis

Statistical analyses were performed using STATA version 8 (StataCorp, College Station, TX, USA). For normally distributed values, Student's *t*-testwas used; otherwise, the nonparametric Mann-Whitney *U*-test was used. For evaluation of all cytokines simultaneously, we used the Hotelling's Tsquare distribution to compare cytokine profiles in the two groups. As the data were not normally distributed, a permutated version of Hotelling's T-square test was done, meaning that all individuals were randomly placed in the two groups 1000 times, and each time *T*2 was calculated. This enables a simulated *p*-value for Hotelling's T-square distribution, not founded on normally distributed values. *p*-Values≤0.05 were considered significant.

### Development of reference ranges for PAPP-A2 between 15 and 40 weeks' gestation

Figure 12 shows the control data from women not diagnosed with preeclampsia at any time during pregnancy and associated 5^{th}, 50^{th} and 95^{th} centiles. These data have been shown to have a varying, but predicable increase in variance after a logarithmic transformation. The median (solid middle line) as well as 90% prediction limits (95^{th} and 5^{th} centiles) are shown (dashed lines). Note that controls were generally distributed into three groups. The early second trimester (approximately 18 to 20 completed weeks' gestation) included 161 observations (7 positive or 4.3% positive), the early third trimester (approximately 27 to 33 weeks' gestation) included 254 observations (11 positive or 4.7% positive) and near term (38 to 40 weeks' gestation) included 100 observations (5 positive or 5.0% positive).

### PAPP-A2 levels in women identified with mild preeclampsia

Figure 13 shows the PAPP-A2 levels by gestational age in pregnancies identified with mild preeclampsia. In the first gestational age group (approximately 18 to 20 completed weeks' gestation) 5 of the 22 observations in cases (23%, the detection rate) are at or above the 95^{th} centile (false positive rate of 5%). In the early third trimester (approximately 27 to 33 weeks' gestation) this rate increases to 23 of 43 (53% detection). Among the 8 near term cases (38 to 40 weeks' gestation only two were elevated (25% detection). From this, PAPP-A2 is best at identifying pregnancies initially indented as having mild preeclampsia in the early third trimester, the time at which clinical symptoms become apparent.

### PAPP-A2 levels in women diagnosed with severe preeclampsia

Figure 14 shows the PAPP-A2 levels by gestational age in pregnancies identified with severe preeclampsia. Often, severe preeclampsia occurs early in pregnancy and requires an early induced delivery. This is why there are no observations after 33 week's gestation. Severe preeclampsia is also less common, indicated by the fewer number of observations in the dataset. In the first gestational age group (approximately 18 to 20 completed weeks' gestation) only 1 of the 7 observations in cases (14%, the detection rate) is at or above the 95^{th} centile (false positive rate of 5%). In the early third trimester (approximately 27 to 33 weeks' gestation) this rate increases to 5 of 11 (45% detection). From this, PAPP-A2 is best at identifying pregnancies initially identified as having severe preeclampsia in the early third trimester.

In conclusion, elevated PAPP-A2 measurements (at or above the 95^{th} centile) occurred in about half of all mild (53%) and severe (45%) preeclampsia early in the third trimester (27 to 33 weeks' gestation).

### Correlations among PAPP-A2, PLGF, sEndoglin, and Activin A levels

The correlation between 1) PLGF and PAPP-A2, 2) sEndoglin and PAPP-A2, 3) 30 PLGF and sEndoglin, 4) sEndoglin and Activin A, and 5) Activin A and PLGF was determined in pregnancy serum from the same cohort of pregnant women described above, which included women with pre-eclampsia (in mild or severe form) and normotensive pregnant women.

Figure 15 illustrates the Spearman rank correlation between PLGF and PAPP-A2 when studied on 604 pregnancy serum samples. This analysis showed no significant correlation between PLGF and PAPP-A2, with a coefficient of -0.18 with a 2-tailed p of <0.0001.

Figure 16 illustrates the Spearman rank correlation between sEndoglin and PAPP-A2 when studied on 211 pregnancy serum samples. This analysis showed a strong correlation between sEndoglin and PAPP-A2 (rs= 0.73, p of <0.0001).

Figure 17 illustrates the Spearman rank correlation between PLGF and sEndoglin 5 when studied on 210 pregnancy serum samples. This analysis showed no significant correlation between PLGF and sEndoglin, with a correlation coefficient of -0.48 with a 2-tailed p of <0.0001.

Figure 18 illustrates the Spearman rank correlation between sEndoglin and Activin A when studied on 211 pregnancy serum samples. This analysis showed a strong correlation between sEndoglin and Activin A (rs= 0.75, p of <0.0001).

Figure 19 illustrates the Spearman rank correlation between Activin A and PLGF when studied on 604 pregnancy serum samples. This analysis showed no significant correlation between Activin A and PLGF, with a correlation coefficient of -0.17 with a 2-tailed p of <0.0001.

The results indicate that both sEndoglin and Activin A, but not PLGF, correlated with PAPP-A2 levels during pregnancy.

## Claims

1. A method to aid in evaluating and/or treating a subject suspected of having or developing preeclampsia, the method comprising the steps of:
(a) determining a level of PAPP-A2 in a sample from the subject; and
(b) comparing said level to a standard level of PAPP-A2 representing the absence of preeclampsia,
wherein the standard level of PAPP-A2 is determined for a gestation time comparable to that of the subject when performing step (a), and wherein the gestation time of the subject, and the gestation time of a population used to determine the standard level value, respectively, are within three weeks of each other;
wherein the standard level of PAPP-A2 is determined as a statistical measure obtained from PAPP-A2 levels of a population of subjects that do not have preeclampsia, and
wherein the statistical measure is a centile level in the range from about the 90th centile to about the 99th centile;
wherein if said level is greater than said standard level the subject is identified as likely to have preeclampsia or to develop preeclampsia, and, if said level is not greater than said standard level, the subject is identified as not likely to have preeclampsia, or not likely to develop preeclampsia.

2. The method of claim 1, wherein the level of a PAPP-A2 polypeptide or a PAPP-A2 nucleic acid is determined as the level of PAPP-A2 in the subject as well as the standard level of PAPP-A2.

3. The method of claim 2, wherein the level of a PAPP-A2 polypeptide is determined using a PAPP-A2-specific antibody.

4. The method of claim 1, wherein the standard level of PAPP-A2 is the 95^{th} centile level.

5. The method of claim 4, wherein the standard level of PAPP-A2 is about 700 ng/ml in plasma at about 39 weeks gestation, or about 350 ng/ml in plasma at about 32 weeks gestation, or about 250 ng/ml in plasma at about 28 weeks gestation, or about 105 ng/ml in plasma at about 19 weeks gestation.

6. The method of claim 1, wherein the gestation time of the subject and the gestation time of the population used to determine the standard value are within one week of each other.

7. The method of claim 1, further comprising:
(c) determining a level in the subject of a secondary marker for preeclampsia selected from the group consisting of activin A, sEndoglin, PAPP-A, PAPP-A-ProMBP and ProMBP-AGT; and
(d) comparing said secondary marker level to a standard level for said secondary marker.

8. The method of claim 7, wherein if said secondary marker level is greater than said secondary marker standard level the subject is identified as more likely to have preeclampsia or to develop preeclampsia than based solely on said determination of PAPP-A2 level.

9. The method of claim 7, wherein if said secondary marker level is not greater than said secondary marker standard level the subject is identified as less likely to have preeclampsia or to develop preeclampsia than based solely on said determination of PAPP-A2 level.

10. The method of claim 7, wherein the secondary marker is activin A or sEndoglin.

11. The method of claim 7, wherein the levels of two of said secondary markers are determined and compared to respective standard levels of said secondary markers.

12. The method of claim 11, wherein if both secondary marker levels are greater than their respective standard levels, the subject is identified as more likely to have preeclampsia or to develop preeclampsia than if only one of the two determined secondary markers is greater than its standard level.

13. The method of claim 11, wherein if both secondary marker levels are not greater than their respective standard levels, the subject is identified as less likely to have preeclampsia or to develop preeclampsia than if both of the two determined secondary markers is greater than its standard level.

14. The method of claim 11, wherein the two secondary markers are activin A and sEndoglin.

15. Use of an assay kit for carrying out the method of any of claims 1 to 14 for diagnosing and/or prognosing pre-eclampsia in a pregnant female human being, said assay kit comprising reagents for determination of the PAPP-A2 level in a biological sample from the pregnant female human being and instructions for use.

16. The use of claim 15, wherein the reagents for determination of the PAPP-A2 level comprises one or more monoclonal and/or polyclonal antibodies specific for PAPP-A2.

## Patentansprüche

1. Verfahren zur Unterstützung bei der Beurteilung und/oder Behandlung einer Person, von der angenommen wird, dass sie eine Präeklampsie hat oder entwickelt, wobei das Verfahren folgende Schritte umfasst:
(a) Bestimmen eines PAPP-A2-Spiegels in einer Probe von der Person, und
(b) Vergleichen des Spiegels mit einem Standard-PAPP-A2-Spiegel, der das Nichtvorliegen einer Präeklampsie darstellt,
wobei der Standard-PAPP-A2-Spiegel für einen Schwangerschaftszeitpunkt bestimmt wird, der mit dem der Person vergleichbar ist, wenn Schritt (a) durchgeführt wird, und wobei der Schwangerschaftszeitpunkt der Person beziehungsweise der Schwangerschaftszeitpunkt einer zur Bestimmung des Werts für den Standardspiegel verwendeten Grundgesamtheit innerhalb von drei Wochen zueinander liegen;
wobei der Standard-PAPP-A2-Spiegel als statistisches Maß bestimmt wird, das aus PAPP-A2-Spiegeln einer Grundgesamtheit von Personen erhalten wird, die keine Präeklampsie haben, und
wobei das statistische Maß ein Perzentil im Bereich von ungefähr des 90. Perzentils bis ungefähr zum 99. Perzentil ist;
wobei festgestellt wird, dass die Person, wenn der Spiegel höher ist als der Standardspiegel, wahrscheinlich eine Präeklampsie hat oder eine Präeklampsie entwickelt, und festgestellt wird, dass die Person, wenn der Spiegel nicht höher ist als der Standardspiegel, wahrscheinlich keine Präeklampsie hat oder wahrscheinlich keine Präeklampsie entwickelt.

2. Verfahren nach Anspruch 1, wobei der Spiegel eines PAPP-A2-Polypeptids oder einer PAPP-A2-Nukleinsäure als PAPP-A2-Spiegel in der Person sowie als Standard-PAPP-A2-Spiegel bestimmt wird.

3. Verfahren nach Anspruch 2, wobei der Spiegel eines PAPP-A2-Polypeptids unter Verwendung eines für PAPP-A2 spezifischen Antikörpers bestimmt wird.

4. Verfahren nach Anspruch 1, wobei der Standard-PAPP-A2-Spiegel das 95. Perzentil ist.

5. Verfahren nach Anspruch 4, wobei der Standard-PAPP-A2-Spiegel ungefähr 700 ng/ml im Plasma in etwa der 39. Schwangerschaftswoche oder ungefähr 350 ng/ml im Plasma in etwa der 32. Schwangerschaftswoche oder ungefähr 250 ng/ml im Plasma in etwa der 28. Schwangerschaftswoche oder ungefähr 105 ng/ml im Plasma in etwa der 19. Schwangerschaftswoche beträgt.

6. Verfahren nach Anspruch 1, wobei der Schwangerschaftszeitpunkt der Person und der Schwangerschaftszeitpunkt der zur Bestimmung des Standardwerts verwendeten Grundgesamtheit innerhalb von einer Woche zueinander liegen.

7. Verfahren nach Anspruch 1, ferner umfassend:
(c) Bestimmen eines Spiegels eines sekundären Markers für Präeklampsie in der Person, der ausgewählt ist aus der Gruppe bestehend aus Aktivin A, sEndoglin, PAPP-A, PAPP-A-ProMBP und ProMBP-AGT; und
(d) Vergleichen des Spiegels des sekundären Markers mit einem Standardspiegel für den sekundären Marker.

8. Verfahren nach Anspruch 7, wobei festgestellt wird, dass es wahrscheinlicher als allein auf der Grundlage der Bestimmung des PAPP-A2-Spiegels ist, dass die Person, wenn der Spiegel des sekundären Markers höher ist als der Standardspiegel des sekundären Markers, eine Präeklampsie hat oder eine Präeklampsie entwickelt.

9. Verfahren nach Anspruch 7, wobei festgestellt wird, dass es weniger wahrscheinlich als allein auf der Grundlage der Bestimmung des PAPP-A2-Spiegels ist, dass die Person, wenn der Spiegel des sekundären Markers nicht höher ist als der Standardspiegel des sekundären Markers, eine Präeklampsie hat oder eine Präeklampsie entwickelt.

10. Verfahren nach Anspruch 7, wobei der sekundäre Marker Aktivin A oder sEndoglin ist.

11. Verfahren nach Anspruch 7, wobei die Spiegel von zwei der sekundären Marker bestimmt und mit jeweiligen Standardspiegeln der sekundären Marker verglichen werden.

12. Verfahren nach Anspruch 11, wobei festgestellt wird, dass die Person, wenn der Spiegel beider sekundärer Marker höher ist als ihre jeweiligen Standardspiegel, mit höherer Wahrscheinlichkeit eine Präeklampsie hat oder eine Präeklampsie entwickelt, als wenn lediglich einer der zwei bestimmten sekundären Marker höher ist als sein Standardspiegel.

13. Verfahren nach Anspruch 11, wobei festgestellt wird, dass die Person, wenn der Spiegel beider sekundärer Marker nicht höher ist als ihre jeweiligen Standardspiegel, mit geringerer Wahrscheinlichkeit eine Präeklampsie hat oder eine Präeklampsie entwickelt, als wenn beide der zwei bestimmten sekundären Marker höher sind als ihr Standardspiegel.

14. Verfahren nach Anspruch 11, wobei die beiden sekundären Marker Aktivin A und sEndoglin sind.

15. Verwendung eines Assay-Kits zum Durchführen des Verfahrens nach einem der Ansprüche 1 bis 14 zur Diagnose und/oder Prognose von Präeklampsie bei einer schwangeren Frau, wobei das Assay-Kit Reagenzien zur Bestimmung des PAPP-A2-Spiegels in einer biologischen Probe von der schwangeren Frau und eine Gebrauchsanweisung umfasst.

16. Verwendung nach Anspruch 15, wobei die Reagenzien zur Bestimmung des PAPP-A2-Spiegels einen oder mehrere monoklonale und/oder polyklonale, für PAPP-A2 spezifische Antikörper umfassen.

## Revendications

1. Procédé pour aider à évaluer et/ou traiter un sujet suspecté d'avoir ou de développer une pré-éclampsie, le procédé comprenant les étapes de :
(a) déterminer un taux de PAPP-A2 dans un échantillon provenant du sujet ; et
(b) comparer ledit taux à un taux standard de PAPP-A2 représentant l'absence de pré-éclampsie,
dans lequel le taux standard de PAPP-A2 est déterminé pour un temps de gestation comparable à celui du sujet lors de la réalisation de l'étape (a), et dans lequel le temps de gestation du sujet, et le temps de gestation d'une population utilisée pour déterminer la valeur du taux standard, respectivement, se situent dans les trois semaines l'un de l'autre,
dans lequel le taux standard de PAPP-A2 est déterminé en tant que mesure statistique obtenue à partir des taux de PAPP-A2 d'une population de sujets qui n'ont pas de pré-éclampsie, et
dans lequel la mesure statistique est un niveau de centile dans la plage d'environ le 90^{ème} centile à environ le 99^{ème} centile ;
dans lequel, si ledit taux est supérieur audit taux standard, le sujet est identifié comme susceptible d'avoir une pré-éclampsie ou de développer une pré-éclampsie, et, si ledit taux n'est pas supérieur audit taux standard, le sujet est identifié comme non susceptible d'avoir une pré-éclampsie ou non susceptible de développer une pré-éclampsie.

2. Procédé selon la revendication 1, dans lequel le taux d'un polypeptide de PAPP-A2 ou d'un acide nucléique de PAPP-A2 est déterminé comme le taux de PAPP-A2 dans le sujet ainsi que le taux standard de PAPP-A2.

3. Procédé selon la revendication 2, dans lequel le taux d'un polypeptide de PAPP-A2 est déterminé à l'aide d'un anticorps spécifique de PAPP-A2.

4. Procédé selon la revendication 1, dans lequel le taux standard de PAPP-A2 est le niveau du 95^{ème} centile.

5. Procédé selon la revendication 4, dans lequel le taux standard de PAPP-A2 est d'environ 700 ng/ml dans le plasma à environ 39 semaines de gestation, ou d'environ 350 ng/ml dans le plasma à environ 32 semaines de gestation, ou d'environ 250 ng/ml dans le plasma à environ 28 semaines de gestation, ou d'environ 105 ng/ml dans le plasma à environ 19 semaines de gestation.

6. Procédé selon la revendication 1, dans lequel le temps de gestation du sujet et le temps de gestation de la population utilisée pour déterminer la valeur standard se situent dans une semaine l'un de l'autre.

7. Procédé selon la revendication 1, comprenant en outre :
(c) déterminer un taux dans le sujet d'un marqueur secondaire pour la pré-éclampsie choisi dans le groupe consistant en l'activine A, la sEndogline, PAPP-A, PAPP-A-ProMBP et ProMBP-AGT ; et
(d) comparer ledit taux de marqueur secondaire à un taux standard pour ledit marqueur secondaire.

8. Procédé selon la revendication 7, dans lequel, si ledit taux de marqueur secondaire est supérieur audit taux standard de marqueur secondaire, le sujet est identifié comme plus susceptible d'avoir une pré-éclampsie ou de développer une pré-éclampsie que sur la base uniquement de ladite détermination du taux de PAPP-A2.

9. Procédé selon la revendication 7, dans lequel, si ledit taux de marqueur secondaire n'est pas supérieur audit taux standard de marqueur secondaire, le sujet est identifié comme moins susceptible d'avoir une pré-éclampsie ou de développer une pré-éclampsie que sur la base uniquement de ladite détermination du taux de PAPP-A2.

10. Procédé selon la revendication 7, dans lequel le marqueur secondaire est l'activine A ou la sEndogline.

11. Procédé selon la revendication 7, dans lequel les taux de deux desdits marqueurs secondaires sont déterminés et comparés à des taux standards respectifs desdits marqueurs secondaires.

12. Procédé selon la revendication 11, dans lequel, si les deux taux de marqueur secondaire sont supérieurs à leurs taux standards respectifs, le sujet est identifié comme plus susceptible d'avoir une pré-éclampsie ou de développer une pré-éclampsie que si uniquement l'un des deux marqueurs secondaires déterminés est supérieur à son taux standard.

13. Procédé selon la revendication 11, dans lequel, si les deux taux de marqueur secondaire ne sont pas supérieurs à leurs taux standards respectifs, le sujet est identifié comme moins susceptible d'avoir une pré-éclampsie ou de développer une pré-éclampsie que si à la fois les deux marqueurs secondaires déterminés sont supérieurs à leur taux standard.

14. Procédé selon la revendication 11, dans lequel les deux marqueurs secondaires sont l'activine A et la sEndogline.

15. Utilisation d'un coffret d'essai pour mettre en oeuvre le procédé de l'une quelconque des revendications 1 à 14 pour le diagnostic et/ou le pronostic d'une pré-éclampsie chez une femme enceinte, ledit coffret d'essai comprenant des réactifs pour la détermination du taux de PAPP-A2 dans un échantillon biologique provenant de la femme enceinte et des instructions pour l'utilisation.

16. Utilisation selon la revendication 15, dans laquelle le réactif pour la détermination du taux de PAPP-A2 comprend un ou plusieurs anticorps monoclonaux et/ou polyclonaux spécifiques pour PAPP-A2.


### SEQUENCE LISTING

<110> Kumar, Ajay, et al.
<120> PAPP-A2 as a marker for monitoring, predicting and diagnosing preeclampsia
<130> 95868
<150> US61/694,991
   <151> 2012-08-30
<160> 2
<170> FastSEQ for Windows Version 4.0
<210> 1
   <211> 1791
   <212> PRT
   <213> Homo sapiens
<400> 1
   Met Met Cys Leu Lys Ile Leu Arg Ile Ser Leu Ala Ile Leu Ala Gly
    1               5                  10                  15
   Trp Ala Leu Cys Ser Ala Asn Ser Glu Leu Gly Trp Thr Arg Lys Lys
               20                  25                  30
   Ser Leu Val Glu Arg Glu His Leu Asn Gln Val Leu Leu Glu Gly Glu
           35                  40                  45
   Arg Cys Trp Leu Gly Ala Lys Val Arg Arg Pro Arg Ala Ser Pro Gln
       50                  55                  60
   His His Leu Phe Gly Val Tyr Pro Ser Arg Ala Gly Asn Tyr Leu Arg
   65                  70                  75                  80
   Pro Tyr Pro Val Gly Glu Gln Glu Ile His His Thr Gly Arg Ser Lys
                   85                  90                  95
   Pro Asp Thr Glu Gly Asn Ala Val Ser Leu Val Pro Pro Asp Leu Thr
               100                 105                 110
   Glu Asn Pro Ala Gly Leu Arg Gly Ala Val Glu Glu Pro Ala Ala Pro
           115                 120                 125
   Trp Val Gly Asp Ser Pro Ile Gly Gln Ser Glu Leu Leu Gly Asp Asp
       130                 135                 140
   Asp Ala Tyr Leu Gly Asn Gln Arg Ser Lys Glu Ser Leu Gly Glu Ala
   145                 150                 155                 160
   Gly Ile Gln Lys Gly Ser Ala Met Ala Ala Thr Thr Thr Thr Ala Ile
                   165                 170                 175
   Phe Thr Thr Leu Asn Glu Pro Lys Pro Glu Thr Gln Arg Arg Gly Trp
               180                 185                 190
   Ala Lys Ser Arg Gln Arg Arg Gln Val Trp Lys Arg Arg Ala Glu Asp
           195                 200                 205
   Gly Gln Gly Asp Ser Gly Ile Ser Ser His Phe Gln Pro Trp Pro Lys
       210                 215                 220
   His Ser Leu Lys His Arg Val Lys Lys Ser Pro Pro Glu Glu Ser Asn
   225                 230                 235                 240
   Gln Asn Gly Gly Glu Gly Ser Tyr Arg Glu Ala Glu Thr Phe Asn Ser
                   245                 250                 255
   Gln Val Gly Leu Pro Ile Leu Tyr Phe Ser Gly Arg Arg Glu Arg Leu
               260                 265                 270
   Leu Leu Arg Pro Glu Val Leu Ala Glu Ile Pro Arg Glu Ala Phe Thr
           275                 280                 285
   Val Glu Ala Trp Val Lys Pro Glu Gly Gly Gln Asn Asn Pro Ala Ile
       290                 295                 300
   Ile Ala Gly Val Phe Asp Asn Cys Ser His Thr Val Ser Asp Lys Gly
   305                 310                 315                 320
   Trp Ala Leu Gly Ile Arg Ser Gly Lys Asp Lys Gly Lys Arg Asp Ala
                   325                 330                 335
   Arg Phe Phe Phe Ser Leu Cys Thr Asp Arg Val Lys Lys Ala Thr Ile
               340                 345                 350
   Leu Ile Ser His Ser Arg Tyr Gln Pro Gly Thr Trp Thr His Val Ala
           355                 360                 365
   Ala Thr Tyr Asp Gly Arg His Met Ala Leu Tyr Val Asp Gly Thr Gln
       370                 375                 380
   Val Ala Ser Ser Leu Asp Gln Ser Gly Pro Leu Asn Ser Pro Phe Met
   385                 390                 395                 400
   Ala Ser Cys Arg Ser Leu Leu Leu Gly Gly Asp Ser Ser Glu Asp Gly
                   405                 410                 415
   His Tyr Phe Arg Gly His Leu Gly Thr Leu Val Phe Trp Ser Thr Ala
               420                 425                 430
   Leu Pro Gln Ser His Phe Gln His Ser Ser Gln His Ser Ser Gly Glu
           435                 440                 445
   Glu Glu Ala Thr Asp Leu Val Leu Thr Ala Ser Phe Glu Pro Val Asn
       450                 455                 460
   Thr Glu Trp Val Pro Phe Arg Asp Glu Lys Tyr Pro Arg Leu Glu Val
   465                 470                 475                 480
   Leu Gln Gly Phe Glu Pro Glu Pro Glu Ile Leu Ser Pro Leu Gln Pro
                   485                 490                 495
   Pro Leu Cys Gly Gln Thr Val Cys Asp Asn Val Glu Leu Ile Ser Gln
               500                 505                 510
   Tyr Asn Gly Tyr Trp Pro Leu Arg Gly Glu Lys Val Ile Arg Tyr Gln
           515                 520                 525
   Val Val Asn Ile Cys Asp Asp Glu Gly Leu Asn Pro Ile Val Ser Glu
       530                 535                 540
   Glu Gln Ile Arg Leu Gln His Glu Ala Leu Asn Glu Ala Phe Ser Arg
   545                 550                 555                 560
   Tyr Asn Ile Ser Trp Gln Leu Ser Val His Gln Val His Asn Ser Thr
                   565                 570                 575
   Leu Arg His Arg Val Val Leu Val Asn Cys Glu Pro Ser Lys Ile Gly
               580                 585                 590
   Asn Asp His Cys Asp Pro Glu Cys Glu His Pro Leu Thr Gly Tyr Asp
           595                 600                 605
   Gly Gly Asp Cys Arg Leu Gln Gly Arg Cys Tyr Ser Trp Asn Arg Arg
       610                 615                 620
   Asp Gly Leu Cys His Val Glu Cys Asn Asn Met Leu Asn Asp Phe Asp
   625                 630                 635                 640
   Asp Gly Asp Cys Cys Asp Pro Gln Val Ala Asp Val Arg Lys Thr Cys
                   645                 650                 655
   Phe Asp Pro Asp Ser Pro Lys Arg Ala Tyr Met Ser Val Lys Glu Leu
               660                 665                 670
   Lys Glu Ala Leu Gln Leu Asn Ser Thr His Phe Leu Asn Ile Tyr Phe
           675                 680                 685
   Ala Ser Ser Val Arg Glu Asp Leu Ala Gly Ala Ala Thr Trp Pro Trp
       690                 695                 700
   Asp Lys Asp Ala Val Thr His Leu Gly Gly Ile Val Leu Ser Pro Ala
   705                 710                 715                 720
   Tyr Tyr Gly Met Pro Gly His Thr Asp Thr Met Ile His Glu Val Gly
                   725                 730                 735
   His Val Leu Gly Leu Tyr His Val Phe Lys Gly Val Ser Glu Arg Glu
               740                 745                 750
   Ser Cys Asn Asp Pro Cys Lys Glu Thr Val Pro Ser Met Glu Thr Gly
           755                 760                 765
   Asp Leu Cys Ala Asp Thr Ala Pro Thr Pro Lys Ser Glu Leu Cys Arg
       770                 775                 780
   Glu Pro Glu Pro Thr Ser Asp Thr Cys Gly Phe Thr Arg Phe Pro Gly
   785                 790                 795                 800
   Ala Pro Phe Thr Asn Tyr Met Ser Tyr Thr Asp Asp Asn Cys Thr Asp
                   805                 810                 815
   Asn Phe Thr Pro Asn Gln Val Ala Arg Met His Cys Tyr Leu Asp Leu
               820                 825                 830
   Val Tyr Gln Gln Trp Thr Glu Ser Arg Lys Pro Thr Pro Ile Pro Ile
           835                 840                 845
   Pro Pro Met Val Ile Gly Gln Thr Asn Lys Ser Leu Thr Ile His Trp
       850                 855                 860
   Leu Pro Pro Ile Ser Gly Val Val Tyr Asp Arg Ala Ser Gly Ser Leu
   865                 870                 875                 880
   Cys Gly Ala Cys Thr Glu Asp Gly Thr Phe Arg Gln Tyr Val His Thr
                   885                 890                 895
   Ala Ser Ser Arg Arg Val Cys Asp Ser Ser Gly Tyr Trp Thr Pro Glu
               900                 905                 910
   Glu Ala Val Gly Pro Pro Asp Val Asp Gln Pro Cys Glu Pro Ser Leu
           915                 920                 925
   Gln Ala Trp Ser Pro Glu Val His Leu Tyr His Met Asn Met Thr Val
       930                 935                 940
   Pro Cys Pro Thr Glu Gly Cys Ser Leu Glu Leu Leu Phe Gln His Pro
   945                 950                 955                 960
   Val Gln Ala Asp Thr Leu Thr Leu Trp Val Thr Ser Phe Phe Met Glu
                   965                 970                 975
   Ser Ser Gln Val Leu Phe Asp Thr Glu Ile Leu Leu Glu Asn Lys Glu
               980                 985                 990
   Ser Val His Leu Gly Pro Leu Asp Thr Phe Cys Asp Ile Pro Leu Thr
           995                 1000                1005
   Ile Lys Leu His Val Asp Gly Lys Val Ser Gly Val Lys Val Tyr Thr
       1010                1015                1020
   Phe Asp Glu Arg Ile Glu Ile Asp Ala Ala Leu Leu Thr Ser Gln Pro
   1025                1030                1035                1040
   His Ser Pro Leu Cys Ser Gly Cys Arg Pro Val Arg Tyr Gln Val Leu
                   1045                1050                1055
   Arg Asp Pro Pro Phe Ala Ser Gly Leu Pro Val Val Val Thr His Ser
               1060                1065                1070
   His Arg Lys Phe Thr Asp Val Glu Val Thr Pro Gly Gln Met Tyr Gln
           1075                1080                1085
   Tyr Gln Val Leu Ala Glu Ala Gly Gly Glu Leu Gly Glu Ala Ser Pro
       1090                1095                1100
   Pro Leu Asn His Ile His Gly Ala Pro Tyr Cys Gly Asp Gly Lys Val
   1105                1110                1115                1120
   Ser Glu Arg Leu Gly Glu Glu Cys Asp Asp Gly Asp Leu Val Ser Gly
                   1125                1130                1135
   Asp Gly Cys Ser Lys Val Cys Glu Leu Glu Glu Gly Phe Asn Cys Val
               1140                1145                1150
   Gly Glu Pro Ser Leu Cys Tyr Met Tyr Glu Gly Asp Gly Ile Cys Glu
           1155                1160                1165
   Pro Phe Glu Arg Lys Thr Ser Ile Val Asp Cys Gly Ile Tyr Thr Pro
       1170                1175                1180
   Lys Gly Tyr Leu Asp Gln Trp Ala Thr Arg Ala Tyr Ser Ser His Glu
   1185                1190                1195                1200
   Asp Lys Lys Lys Cys Pro Val Ser Leu Val Thr Gly Glu Pro His Ser
                   1205                1210                1215
   Leu Ile Cys Thr Ser Tyr His Pro Asp Leu Pro Asn His Arg Pro Leu
               1220                1225                1230
   Thr Gly Trp Phe Pro Cys Val Ala Ser Glu Asn Glu Thr Gln Asp Asp
           1235                1240                1245
   Arg Ser Glu Gln Pro Glu Gly Ser Leu Lys Lys Glu Asp Glu Val Trp
       1250                1255                1260
   Leu Lys Val Cys Phe Asn Arg Pro Gly Glu Ala Arg Ala Ile Phe Ile
   1265                1270                1275                1280
   Phe Leu Thr Thr Asp Gly Leu Val Pro Gly Glu His Gln Gln Pro Thr
                   1285                1290                1295
   Val Thr Leu Tyr Leu Thr Asp Val Arg Gly Ser Asn His Ser Leu Gly
               1300                1305                1310
   Thr Tyr Gly Leu Ser Cys Gln His Asn Pro Leu Ile Ile Asn Val Thr
           1315                1320                1325
   His His Gln Asn Val Leu Phe His His Thr Thr Ser Val Leu Leu Asn
       1330                1335                1340
   Phe Ser Ser Pro Arg Val Gly Ile Ser Ala Val Ala Leu Arg Thr Ser
   1345                1350                1355                1360
   Ser Arg Ile Gly Leu Ser Ala Pro Ser Asn Cys Ile Ser Glu Asp Glu
                   1365                1370                1375
   Gly Gln Asn His Gln Gly Gln Ser Cys Ile His Arg Pro Cys Gly Lys
               1380                1385                1390
   Gln Asp Ser Cys Pro Ser Leu Leu Leu Asp His Ala Asp Val Val Asn
           1395                1400                1405
   Cys Thr Ser Ile Gly Pro Gly Leu Met Lys Cys Ala Ile Thr Cys Gln
       1410                1415                1420
   Arg Gly Phe Ala Leu Gln Ala Ser Ser Gly Gln Tyr Ile Arg Pro Met
   1425                1430                1435                1440
   Gln Lys Glu Ile Leu Leu Thr Cys Ser Ser Gly His Trp Asp Gln Asn
                   1445                1450                1455
   Val Ser Cys Leu Pro Val Asp Cys Gly Val Pro Asp Pro Ser Leu Val
               1460                1465                1470
   Asn Tyr Ala Asn Phe Ser Cys Ser Glu Gly Thr Lys Phe Leu Lys Arg
           1475                1480                1485
   Cys Ser Ile Ser Cys Val Pro Pro Ala Lys Leu Gln Gly Leu Ser Pro
       1490                1495                1500
   Trp Leu Thr Cys Leu Glu Asp Gly Leu Trp Ser Leu Pro Glu Val Tyr
   1505                1510                1515                1520
   Cys Lys Leu Glu Cys Asp Ala Pro Pro Ile Ile Leu Asn Ala Asn Leu
                   1525                1530                1535
   Leu Leu Pro His Cys Leu Gln Asp Asn His Asp Val Gly Thr Ile Cys
               1540                1545                1550
   Lys Tyr Glu Cys Lys Pro Gly Tyr Tyr Val Ala Glu Ser Ala Glu Gly
           1555                1560                1565
   Lys Val Arg Asn Lys Leu Leu Lys Ile Gln Cys Leu Glu Gly Gly Ile
       1570                1575                1580
   Trp Glu Gln Gly Ser Cys Ile Pro Val Val Cys Glu Pro Pro Pro Pro
   1585                1590                1595                1600
   Val Phe Glu Gly Met Tyr Glu Cys Thr Asn Gly Phe Ser Leu Asp Ser
                   1605                1610                1615
   Gln Cys Val Leu Asn Cys Asn Gln Glu Arg Glu Lys Leu Pro Ile Leu
               1620                1625                1630
   Cys Thr Lys Glu Gly Leu Trp Thr Gln Glu Phe Lys Leu Cys Glu Asn
           1635                1640                1645
   Leu Gln Gly Glu Cys Pro Pro Pro Pro Ser Glu Leu Asn Ser Val Glu
       1650                1655                1660
   Tyr Lys Cys Glu Gln Gly Tyr Gly Ile Gly Ala Val Cys Ser Pro Leu
   1665                1670                1675                1680
   Cys Val Ile Pro Pro Ser Asp Pro Val Met Leu Pro Glu Asn Ile Thr
                   1685                1690                1695
   Ala Asp Thr Leu Glu His Trp Met Glu Pro Val Lys Val Gln Ser Ile
               1700                1705                1710
   Val Cys Thr Gly Arg Arg Gln Trp His Pro Asp Pro Val Leu Val His
           1715                1720                1725
   Cys Ile Gln Ser Cys Glu Pro Phe Gln Ala Asp Gly Trp Cys Asp Thr
       1730                1735                1740
   Ile Asn Asn Arg Ala Tyr Cys His Tyr Asp Gly Gly Asp Cys Cys Ser
   1745                1750                1755                1760
   Ser Thr Leu Ser Ser Lys Lys Val Ile Pro Phe Ala Ala Asp Cys Asp
                   1765                1770                1775
   Leu Asp Glu Cys Thr Cys Arg Asp Pro Lys Ala Glu Glu Asn Gln
               1780                1785                1790
<210> 2
   <211> 5376
   <212> DNA
   <213> Homo sapiens
<400> 2
   atgatgtgct taaagatcct aagaataagc ctggcgattt tggctgggtg ggcactctgt 60
   tctgccaact ctgagctggg ctggacacgc aagaaatcct tggttgagag ggaacacctg 120
   aatcaggtgc tgttggaagg agaacgttgt tggctggggg ccaaggttcg aagacccaga 180
   gcttctccac agcatcacct ctttggagtc taccccagca gggctgggaa ctacctaagg 240
   ccctaccccg tgggggagca agaaatccat catacaggac gcagcaaacc agacactgaa 300
   ggaaatgctg tgagccttgt tcccccagac ctgactgaaa atccagcagg actgaggggt 360
   gcagttgaag agccggctgc cccatgggta ggggatagtc ctattgggca atctgagctg 420
   ctgggagatg atgacgctta tctcggcaat caaagatcca aggagtctct aggtgaggcc 480
   gggattcaga aaggctcagc catggctgcc actactacca ccgccatttt cacaaccctg 540
   aacgaaccca aaccagagac ccaaaggagg ggctgggcca agtccaggca gcgtcgccaa 600
   gtgtggaaga ggcgggcgga agatgggcag ggagactccg gtatctcttc acatttccaa 660
   ccttggccca agcattccct taaacacagg gtcaaaaaga gtccaccgga ggaaagcaac 720
   caaaatggtg gagagggctc ctaccgagaa gcagagacct ttaactccca agtaggactg 780
   cccatcttat acttctctgg gaggcgggag cggctgctgc tgcgtccaga agtgctggct 840
   gagattcccc gggaggcgtt cacagtggaa gcctgggtta aaccggaggg aggacagaac 900
   aacccagcca tcatcgcagg tgtgtttgat aactgctccc acactgtcag tgacaaaggc 960
   tgggccctgg ggatccgctc agggaaggac aagggaaagc gggatgctcg cttcttcttc 1020
   tccctctgca ccgaccgcgt gaagaaagcc accatcttga ttagccacag tcgctaccaa 1080
   ccaggcacat ggacccatgt ggcagccact tacgatggac ggcacatggc cctgtatgtg 1140
   gatggcactc aggtggctag cagtctagac cagtctggtc ccctgaacag ccccttcatg 1200
   gcatcttgcc gctctttgct cctgggggga gacagctctg aggatgggca ctatttccgt 1260
   ggacacctgg gcacactggt tttctggtcg accgccctgc cacaaagcca ttttcagcac 1320
   agttctcagc attcaagtgg ggaggaggaa gcgactgact tggtcctgac agcgagcttt 1380
   gagcctgtga acacagagtg ggttcccttt agagatgaga agtacccacg acttgaggtt 1440
   ctccagggct ttgagccaga gcctgagatt ctgtcgcctt tgcagccccc actctgtggg 1500
   caaacagtct gtgacaatgt ggaattgatc tcccagtaca atggatactg gccccttcgg 1560
   ggagagaagg tgatacgcta ccaggtggtg aacatctgtg atgatgaggg cctaaacccc 1620
   attgtgagtg aggagcagat tcgtctgcag cacgaggcac tgaatgaggc cttcagccgc 1680
   tacaacatca gctggcagct gagcgtccac caggtccaca attccaccct gcgacaccgg 1740
   gttgtgcttg tgaactgtga gcccagcaag attggcaatg accattgtga ccccgagtgt 1800
   gagcacccac tcacaggcta tgatgggggt gactgccgcc tgcagggccg ctgctactcc 1860
   tggaaccgca gggatgggct ctgtcacgtg gagtgtaaca acatgctgaa cgactttgac 1920
   gacggagact gctgcgaccc ccaggtggct gatgtgcgca agacctgctt tgaccctgac 1980
   tcacccaaga gggcatacat gagtgtgaag gagctgaagg aggccctgca gctgaacagt 2040
   actcacttcc tcaacatcta ctttgccagc tcagtgcggg aagaccttgc aggtgctgcc 2100
   acctggcctt gggacaagga cgctgtcact cacctgggtg gcattgtcct cagcccagca 2160
   tattatggga tgcctggcca caccgacacc atgatccatg aagtgggaca tgttctggga 2220
   ctctaccatg tctttaaagg agtcagtgaa agagaatcct gcaatgaccc ctgcaaggag 2280
   acagtgccat ccatggaaac gggagacctc tgtgccgaca ccgcccccac tcccaagagt 2340
   gagctgtgcc gggaaccaga gcccactagt gacacctgtg gcttcactcg cttcccaggg 2400
   gctccgttca ccaactacat gagctacacg gatgataact gcactgacaa cttcactcct 2460
   aaccaagtgg cccgaatgca ttgctatttg gacctagtct atcagcagtg gactgaaagc 2520
   agaaagccca cccccatccc cattccacct atggtcatcg gacagaccaa caagtccctc 2580
   actatccact ggctgcctcc tattagtgga gttgtatatg acagggcctc aggcagcttg 2640
   tgtggcgctt gcactgaaga tgggaccttt cgtcagtatg tgcacacagc ttcctcccgg 2700
   cgggtgtgtg actcctcagg ttattggacc ccagaggagg ctgtggggcc tcctgatgtg 2760
   gatcagccct gcgagccaag cttacaggcc tggagccctg aggtccacct gtaccacatg 2820
   aacatgacgg tcccctgccc cacagaaggc tgtagcttgg agctgctctt ccaacacccg 2880
   gtccaagccg acaccctcac cctgtgggtc acttccttct tcatggagtc ctcgcaggtc 2940
   ctctttgaca cagagatctt gctggaaaac aaggagtcag tgcacctggg ccccttagac 3000
   actttctgtg acatcccact caccatcaaa ctgcacgtgg atgggaaggt gtcgggggtg 3060
   aaagtctaca cctttgatga gaggatagag attgatgcag cactcctgac ttctcagccc 3120
   cacagtccct tgtgctctgg ctgcaggcct gtgaggtacc aggttctccg cgatccccca 3180
   tttgccagtg gtttgcccgt ggtggtgaca cattctcaca ggaagttcac ggacgtggag 3240
   gtcacacctg gacagatgta tcagtaccaa gttctagctg aagctggagg agaactggga 3300
   gaagcttcgc ctcctctgaa ccacattcat ggagctcctt attgtggaga tgggaaggtg 3360
   tcagagagac tgggagaaga gtgtgatgat ggagaccttg tgagcggaga tggctgctcc 3420
   aaggtgtgtg agctggagga aggtttcaac tgtgtaggag agccaagcct ttgctacatg 3480
   tatgagggag atggcatatg tgaacctttt gagagaaaaa ccagcattgt agactgtggc 3540
   atctacactc ccaaaggata cttggatcaa tgggctaccc gggcttactc ctctcatgaa 3600
   gacaagaaga agtgtcctgt ttccttggta actggagaac ctcattccct aatttgcaca 3660
   tcataccatc cagatttacc caaccaccgt cccctaactg gctggtttcc ctgtgttgcc 3720
   agtgaaaatg aaactcagga tgacaggagt gaacagccag aaggtagcct gaagaaagag 3780
   gatgaggttt ggctcaaagt gtgtttcaat agaccaggag aggccagagc aatttttatt 3840
   tttttgacaa ctgatggcct agttcccgga gagcatcagc agccgacagt gactctctac 3900
   ctgaccgatg tccgtggaag caaccactct cttggaacct atggactgtc atgccagcat 3960
   aatccactga ttatcaatgt gacccatcac cagaatgtcc ttttccacca taccacctca 4020
   gtgctgctga atttctcatc cccacgggtc ggcatctcag ctgtggctct aaggacatcc 4080
   tcccgcattg gtctttcggc tcccagtaac tgcatctcag aggacgaggg gcagaatcat 4140
   cagggacaga gctgtatcca tcggccctgt gggaagcagg acagctgtcc gtcattgctg 4200
   cttgatcatg ctgatgtggt gaactgtacc tctataggcc caggtctcat gaagtgtgct 4260
   atcacttgtc aaaggggatt tgcccttcag gccagcagtg ggcagtacat caggcccatg 4320
   cagaaggaaa ttctgctcac atgttcttct gggcactggg accagaatgt gagctgcctt 4380
   cccgtggact gcggtgttcc cgacccgtct ttggtgaact atgcaaactt ctcctgctca 4440
   gagggaacca aatttctgaa acgctgctca atctcttgtg tcccaccagc caagctgcaa 4500
   ggactgagcc catggctgac atgtcttgaa gatggtctct ggtctctccc tgaagtctac 4560
   tgcaagttgg agtgtgatgc tccccctatt attctgaatg ccaacttgct cctgcctcac 4620
   tgcctccagg acaaccacga cgtgggcacc atctgcaaat atgaatgcaa accagggtac 4680
   tatgtggcag aaagtgcaga gggtaaagtc aggaacaagc tcctgaagat acaatgcctg 4740
   gaaggtggaa tctgggagca aggcagctgc attcctgtgg tgtgtgagcc accccctcct 4800
   gtgtttgaag gcatgtatga atgtaccaat ggcttcagcc tggacagcca gtgtgtgctc 4860
   aactgtaacc aggaacgtga aaagcttccc atcctctgca ctaaagaggg cctgtggacc 4920
   caggagttta agttgtgtga gaatctgcaa ggagaatgcc caccaccccc ctcagagctg 4980
   aattctgtgg agtacaaatg tgaacaagga tatgggattg gtgcagtgtg ttccccattg 5040
   tgtgtaatcc cccccagtga ccccgtgatg ctacctgaga atatcactgc tgacactctg 5100
   gagcactgga tggaacctgt caaagtccag agcattgtgt gcactggccg gcgtcaatgg 5160
   cacccagacc ccgtcttagt ccactgcatc cagtcatgtg agcccttcca agcagatggt 5220
   tggtgtgaca ctatcaacaa ccgagcctac tgccactatg acgggggaga ctgctgctct 5280
   tccacactct cctccaagaa ggtcattcca tttgctgctg actgtgacct ggatgagtgc 5340
   acctgccggg accccaaggc agaagaaaat cagtaa                           5376